# EUROPEAN PATENT SPECIFICATION

(11) **EP 4 228 562 B1**
(45) Date of publication and mention of the grant of the patent: **07.01.2026**
(21) Application number: 21890440.7
(22) Date of filing: 12.11.2021
(51) Int. Cl.: A61F 5/01, A61F 5/052

(54) **ENERGY STORING KNEE BRACE**
ENERGIESPEICHERNDE KNIESTÜTZE
GENOUILLÈRE À ACCUMULATION D'ÉNERGIE

(30) Priority: 13.11.2020 US 202063113797 P
(43) Date of publication of application: 23.08.2023
(73) Proprietor: Spring Loaded Technology Incorporated, Dartmouth, NS B3B 1T2 (CA)
(72) Inventor: ERIC MACKEIL, Bradley, Fletchers Lake, Nova Scotia B2T 1A5 (CA); NATHANIEL DONALD ELLSMERE, Joseph, Halifax, Nova Scotia B3J 1Y3 (CA); BRUCE FITZGERALD, Stephen, Halifax, Nova Scotia B3M 2V1 (CA); DAVID COWPER-SMITH, Christopher, Halifax, Ontario B3L 1J7 (CA); PAULA KEREKES, Deanna, Dartmouth, Nova Scotia B3A 0G6 (CA); CHRISTOPHER BURNS, Evan, Lunenburg, Nova Scotia B0J 2C0 (CA); MARK PARKHILL, Andrew, Halifax, Nova Scotia B3H 4M9 (CA); ALLEN HARRIS, William, Bedford, Nova Scotia B4A 3C4 (CA)
(74) Representative: Marks & Clerk LLP
(86) International application number: PCT/CA2021/051608
(87) International publication number: WO 2022/099416

(56) References cited:
- WO-A1-2017/041165
- US-A- 5 395 304
- US-A1- 2015 119 777
- US-A1- 2015 374 530
- US-B2- 9 844 454

## Description

### FIELD

In one of its aspects, the present disclosure relates generally to braces for limbs, and in particular braces that that include an energy storage system (such as a spring) that can store energy when the brace is flexed and can then apply a restorative force that urges the brace to return to its extended configuration.

### BACKGROUND

United States Patent no. 9,416,838 (Garrish) discloses a hydraulic tension spring comprises a block comprising at least one liquid-impermeable cylinder and at least one piston disposed through an open end of the cylinder. The piston provides a piston guide within a liquid containment space within the cylinder filled with hydraulic fluid. A tensioning member moves the piston or the cylinder relative to the other to compress the hydraulic fluid and load the spring, while the piston guide keeps the piston axially aligned to prevent buckling.

United States Patent no. 10,070,983 (Garrish) discloses a hinge for a brace that includes a tensioning element such as an inelastic cord for applying a restorative force to the hinge. A tensioning mechanism, preferably a hydraulic spring, tensions the cord as the hinge is moved from a rest position to a loaded condition. A tensioning member having a peripheral cam surface applies a tensioning force to the cord as the hinge is moved out of the rest position. The tensioning block and the anchoring block may be operatively geared together along an arc of motion so as to provide a generally symmetrical motion of the tensioning and anchoring portions relative to the gusset. When an external force is applied to move the hinge from the rest position the tension on the cord loads the spring, and when the external force is removed the spring applies a restorative force to move the hinge back to the rest position. Optionally the tensioning member may be configured to be disengaged and reengaged by an external actuator.

United States Patent Publication no. 2015/0374532 (Fendon) discloses an orthopedic brace for a body joint has a first longitudinal support, a second longitudinal support and a hinge joint rotatably connecting the longitudinal supports, thereby rendering them positionable at different hinge joint angles. The brace also has a tensioner attached at its opposite ends to the first and second longitudinal supports which intermediately engages a cam positioned at the hinge joint. The tensioner applies a variable elastic tensioning force to the longitudinal supports which varies as a function of the hinge joint angle and biases the longitudinal supports toward a position corresponding to a desired hinge joint angle.

United States Patent Publication no. 2018/0078399 (Garrish) discloses a brace for augmenting movement of a user's limb about a joint includes an upper arm having an engaging portion for engaging against the user's limb above the joint, and an attachment portion, a lower arm having an engaging portion for engaging against the user's limb below the joint, and an attachment portion, the lower arm being pivotable relative to the upper arm, at least one compression element disposed in fixed relation to at least one of the upper and lower arms, and a substantially inelastic tensioning element affixed to the other of the upper and lower arms over at least one tensioning element tensioning member such that a user applies a force to move the brace to a loaded position in which the at least one compression element is loaded and upon removal of the applied force the at least one compression element applies a restoring force to urge the brace out of the loaded position, the at least one compression element comprising a hydraulic tension spring disposed in fixed relation to at least the other of the upper and lower arms, comprising a pair of cylinders mounted within a frame, each cylinder having a sealed portion defining a liquid containment space, for each of the pair of cylinders, a piston comprising a piston rod, the piston rod comprising a compressing portion having a smaller diameter than the cylinder and extending axially through a hydraulic seal into the liquid containment space, and an external portion accessible from outside the liquid containment space, one of the cylinder and the piston being fixed relative to the frame and the other of the cylinder and the piston being movable axially relative to the frame, a guide for maintaining the movable one of the cylinder and the piston oriented axially relative to the frame, and a compression element tensioning member bearing against the movable one of the at least one cylinder and the piston, for compressing the at least one cylinder relative to the piston, whereby when the frame is fixed in place and tension is applied to the tensioning element tensioning member, the compressing portions of the piston rods intrude further into the liquid containment space, compressing the hydraulic fluid and loading the spring.

U.S. Patent Publication No. 2015/0005685 (Ram-srinivasa) discloses a dynamic tension system that connects to an orthopedic device having a frame and a hinge connected to the frame. The dynamic tension system includes a cable, an adjustment mechanism connected to the cable and arranged to incrementally wind or release the cable, and a tension control device connected to the adjustment mechanism and arranged to limit the adjustment mechanism from winding of the cable past a predetermined tension level.

U.S. Patent Publication no. 2021/0205111 (Johnson) discloses an adjustable tension knee brace for unloading weight from a knee joint afflicted with osteoarthritis, thus reducing pain and improving mobility, comprising: an upper and lower frame connected by an unloading hinge assembly, optionally comprising a sensor and processor allowing for remote or automatic control of brace tension. In embodiments, the brace includes a user mechanism that is capable of adjusting a tensioning element while the brace is being worn. In other embodiments, electronic motors, sensors, and indicators may be included in the brace to improve brace performance and user interaction.

U.S. Patent Publication no. 2015119777A1 (Garrish) discloses a brace for augmenting extension of a user's limb about a joint comprises an upper arm pivotably attached to a lower arm. At least one compression element is attached to at least one of the upper and lower arms, and a substantially inelastic tensioning element is affixed to the other of the upper and lower arms over at least one tensioning member. Applying a force to bend the brace to a flexed position loads the compression element, and upon removal of the flexing force the compression element applies a restoring force to urge the brace back to the extended position. The compression element may comprise a hydraulic tension spring comprising at least one cylinder having a sealed portion defining a liquid containment space, and a piston comprising a compressing portion having a smaller diameter than the cylinder and extending axially through a hydraulic seal into the liquid containment space. A guide for maintaining the piston oriented axially relative to the frame maintains piston alignment so that when the frame is fixed in place and tension is applied to the tensioning member, the compressing portion of the piston rod intrudes further into the liquid containment space, compressing the hydraulic fluid and loading the spring. US 2015/119777A1 discloses an energy storing knee brace according to the preamble of claim 1.

### SUMMARY

A knee brace can perform a purely prophylactic function, or provide an assistive force that helps the user to extend their knee, or both. Knee braces can provide physical protection against injury, and may for example be used by athletes involved in high-risk sports where there is a relatively high susceptibility to sustaining a knee injury.

Many individuals suffer from knee problems, often due to a prior knee injury. Some such problems can significantly affect mobility and/or the ability to support the injured person. While corrective measures such as exercise and physiotherapy, or in more serious cases surgery, can assist in correcting or partially alleviating some knee problems, there remains a need in many cases for knee support and extension augmentation.

Particularly where there has been ligament damage, for example a tear or strain in the anterior cruciate ligament (ACL), posterior cruciate ligament (PCL), medial collateral ligament (MCL) or lateral collateral ligament (LCL), a knee brace can be used to both provide support and enhance extension strength, and thus reduce the load on the injured knee. Conventional knee braces that provide active assistance to knee extension are designed to yield when the knee is flexed, loading a torsion spring or compression spring in the process. The spring is loaded when the user bends their leg, and when extending their leg the spring unloads applying a force that augments the extension action. This also helps to support the user and prevent collapse if the injured knee buckles.

In some conventional knee braces the brace includes an upper frame that is configured to receive and be attached to the thigh of a user (e.g. above the knee joint) and a lower frame upper frame that is configured to receive and be attached to the calf or lower leg of the user (e.g. on the lower side of the knee). Each frame can include a pair of longitudinally extending arms that extend along the sides of the user's leg, and at least one cross-member that extends laterally between the arms and helps define the shape of the frames. The upper and lower frames are connected to each other using at least one hinge, and usually by a pair of hinges that are located on opposite sides of the user's knee.

In conventional braces that include some type of spring mechanism that is loaded when the brace is flexed, the energy storage mechanisms e.g. springs or other elastic members, are located within the hinges or are generally located within one of the longitudinally extending arms so that the energy storage mechanism is located close to the hinge. For example, U.S. patent no. 10,070,983 discloses a hinge for a brace in which a hydraulic spring is located within the arm of the upper brace frame, adjacent the hinge. An inelastic tensioning cord passes over the hinge and is engage by the hinge when the brace is flexed, thereby apply a tension to the spring and loading the spring. Locating the spring adjacent the hinge reduces the length of the tensioning cord, avoids the need for the cord to change direction, and simplifies the mounting of the spring.

In a contrasting example, U.S. patent publication no. 2021/0205111 discloses a brace in which the upper and lower frames are connected using hinges with intermeshing gears and in which an elastic tensioning element is stretched across the hinges when the brace is flexed. In this arrangement, the tensioning element is elastic and functions as the energy storage member, instead of having an inelastic cord connected to a separate spring. The US '111 publication positions the elastic cord within the arms of the brace frames and in a position where it directly passes over and engages the hinges when in use.

In such configurations, the structural portions of the brace frame are subjected to bending forces when in use and carry substantial portions of the restorative force that is created or generated by the spring assembly. To help carry and resist such loads, the frame of the brace is formed from a material that is strong enough and stiff enough to resist deflection or failure when loaded. For example, the existing Levitation 2 knee brace manufactured by Spring Loaded Technologies of Nova Scotia, Canada uses a single "powered" (spring actuated) hinge, and one passive unpowered hinge. The unpowered hinge's purpose is to help the brace track the motion of the knee, and to help provide lateral stiffness to the brace frame, but not to provide joint unloading forces. This, in effect, means the majority of the forces and stresses the brace carries to support a user's weight and unload the knee are routed through one side of the brace. The hinge, therefore, must be made from a strong alloy, such as aluminum, to withstand these forces. This can require a relatively expensive and time-consuming manufacturing process. Moreover, this single-sided configuration necessitates a very stiff brace frame to carry torsional loads. That is, braces having such a configuration may tend to generates a restorative torque which linearly increases with the flexion angle of the hinge. The slope of the Torque-Flexion Angle graph of this hinge (i.e. the stiffness constant) is a fixed value. Utilizing a tension adjustment mechanism in the such designs hinge can alter the starting angle at which the restorative force begins, but cannot alter the stiffness constant.

If a brace is configured to generate a relatively high restorative force, such as the braces shown in U.S. patent no. 10,070,983 the frame of the brace needs to be formed from a sufficiently strong material, such as metal, composites, carbon fiber and the like. While such materials are strong, they can be challenging to manufacture, can have relatively high costs and can relatively difficult to form into frame designs that are comfortable and well-fitted for a user.

Locating the energy storage members, such as springs, etc. close to the hinges also requires that the arms of the frames be sized and shaped to accommodate the spring structure. This can often mean that the arms, or at least one arm that includes the spring mechanism, are configured to be relatively wider than may otherwise be possible in order to accommodate the spring mechanism and related hardware, etc. This can increase the overall size of the brace, and in particular the arms that are adjacent the user's leg, which can make the brace feel uncomfortable or may interfere with wearing clothes over the brace or other aspects of the user's daily life.

Locating a spring member within or mounted to the arms of the brace frame may also limit how the arm can be shaped. For example, the arms shown in U.S. patent no. 10,070,983 are configured to have a generally straight section adjacent the hinge so that they can accommodate the volume and linear range of motion of the hydraulic spring that is mounted within the arms. This can make it difficult for the arms to be shaped to follow the shape or contours of the user's leg.

Therefore, there remains a need for a brace, such as a knee brace, that can include an energy storage assembly containing at least one spring member that is configured to be loaded when the brace is flexed and to apply a desired restorative force, but in which the spring member is remote and/or physically spaced part from the hinges. Locating the spring member, and preferably other portions of the energy storage assembly in a location that is remote from the hinges may allow the arms of the brace frames to have a relatively smaller overall size and to have a more preferred shape that is not dictated by the size or operational requirements of the spring assembly.

Preferably, the spring member can be longitudinally offset from the hinges, and may be provided toward the upper (or optionally lower) end of the brace. The spring member may also be laterally offset from the arms, and preferably is located laterally between the arms of the brace, such that the spring member will overlie the upper leg, or alternatively the lower leg, of the user. Optionally, the spring member can be mounted to the laterally extending cross member of the upper frame section, so that the spring member is registered on the front side of the user's upper leg. In this location, the spring assembly does not extend the overall width of the brace in the lateral direction and allows for relatively smaller arms to be provided.

To transmit forces between the spring member and the hinges, the braces described herein can include a substantially inelastic and flexible tensioning cord that can travel along/within a suitable cord path from the hinge(s) to the energy storage assembly.

The cord path as described herein can include at least one longitudinally extending portion that extends from the hinge in the direction of the arm of the upper frame (and preferably within the arm). This section need not be linear, but can be understood to extend substantially in the longitudinal direction from the hinge to a location proximate the lateral cross-member. The cord path then also includes a generally laterally extending portion that travels in the direction of the cross-member (and preferably within the cross-member), such that the tension force is transferred laterally from the arm, across the cross-member and to the spring member.

Optionally, the spring member can be grounded on the frame, such that the frame provides most, if not all of the reaction force to balance the force generated by the spring member. This can help simplify the design of the brace and the path of the flexible tension cord. However, this design can require a relatively strong brace frame to withstand the restorative force loading. A brace made using this design may have frames made of metal, carbon fiber, composite materials, strong or reinforced plastic or the like.

Alternatively, the energy storage assembly may be configured to support the spring member such that the spring number is not grounded on the frame, and instead where the spring force is entirely carried by the tension cord. In such examples, the tension cord may be formed in a loop, or bight, that surrounds the spring member. Tension exerted on the cord can then cause the loop/bight to constrict and squeeze both opposing ends of the spring member. If the spring member is arranged as a compression spring, such as a hydraulic compression spring, the forces exerted by the spring member can act on the tensioning cord to apply the restorative force without having the spring member directly grounded on the frame. By configuring the system so that the relatively strong tensioning cord carries all, or at least substantially all of the spring/restorative forces, the frame may be made from relatively weaker material. This may allow the frame to be made from relatively weaker material, such as plastics and the like, which may simplify manufacturing. This may also allow portions of the frame to be manufactured using techniques, such as additive manufacturing/ 3D printing which can be used to make relatively complicated shapes from plastics, but would not be usable if the frame were to be formed from carbon fiber, metal and other such materials.

The teachings described herein, in one broad aspect, relate to an energy storing knee brace to be worn on a leg according to independent claim 1, with optional features set out in the dependent claims.

Other advantages of the present teachings may become apparent to those of skill in the art upon reviewing the present specification.

### BRIEF DESCRIPTION OF THE DRAWINGS

Embodiments of the present invention will be described with reference to the accompanying drawings, wherein like reference numerals denote like parts, and in which:
Figure 1 is a perspective view of one example of a brace in an extended position;
Figure 2 is a perspective view of the brace of Figure 1 in an intermediate, partially flexed position;
Figure 3 is a perspective view of the brace of Figure 1 in a flexed position;
Figure 4 is a front view of the brace of Figure 1 with the interior cord and spring member revealed;
Figure 5 is a front view of another example of a brace with the interior cord and spring member revealed;
Figure 6 is a side view of a hinge in an extended position with the near outer link removed;
Figure 7 is a side view of the hinge of Figure 6 in an intermediate, partially flexed position;
Figure 8 is a side view of a hinge of Figure 6 in a flexed position;
Figure 9 is a side view of the hinge of Figure 6 in an extended position with the near outer link and an inner link removed;
Figure 10 is a side view of the hinge of Figure 9 in an intermediate, partially flexed position;
Figure 11 is a side view of a hinge of Figure 9 in a flexed position;
Figure 12 is an isolated view of portions of the tensioning cord and energy storage assembly of the brace of Figure 1, corresponding to an extended position;
Figure 13 is an isolated view of portions of the tensioning cord and energy storage assembly of the brace of Figure 1, corresponding to an intermediate, partially flexed position;
Figure 14 is an isolated view of portions of the tensioning cord and energy storage assembly of the brace of Figure 1, corresponding to a flexed position;
Figure 15 is a schematic view of another example of a brace;
Figure 16 is a schematic view of another example of a brace;
Figure 17 is a schematic view of another example of a brace;
Figure 18 is a schematic view of another example of a brace;
Figure 19 is a photograph of another example of a brace;
Figure 20 is a side view of the brace of Figure 19;
Figure 21 is a perspective view of the brace of Figure 19;
Figure 22 is a perspective view of a portion of the brace of Figure 19;
Figure 23 is a view of portions of the brace of Figure 19 in isolation;
Figure 24 is a front view of another example of a brace;
Figure 25 is an enlarged view of a portion of the brace of Figure 24;
Figure 26 is a front perspective view of another example of a brace in its extended position;
Figure 27 is a front perspective view of the brace of Figure 26 in an intermediate, partially flexed position;
Figure 28 is a view of the brace of Figure 26 in an intermediate, partially flexed position; and
Figure 29 is a front perspective view of the brace of Figure 26 in a flexed position.

### DETAILED DESCRIPTION

Various apparatuses or processes will be described below to provide an example of an embodiment of each claimed invention. No embodiment described below limits any claimed invention and any claimed invention may cover processes or apparatuses that differ from those described below. The claimed inventions are not limited to apparatuses or processes having all of the features of any one apparatus or process described below or to features common to multiple or all of the apparatuses described below. It is possible that an apparatus or process described below is not an embodiment of any claimed invention. Any invention disclosed in an apparatus or process described below that is not claimed in this document may be the subject matter of another protective instrument, for example, a continuing patent application, and the applicants, inventors or owners do not intend to abandon, disclaim, or dedicate to the public any such invention by its disclosure in this document.

The teachings herein relate generally to braces for limbs, and in particular braces that that include an energy storage system (such as a spring) that can store energy when the brace is flexed and can then apply a restorative force that urges the brace to return to its extended configuration. One example of such braces are knee braces that can be worn on the leg of a human user. Another example is elbow braces that are worn on the arm of a user, and possibly braces configured to accommodate other joints of either human or animal users. For the purposes of illustrating some of the features that can be used in combination with any suitable brace, the present specification includes illustrations and descriptions of some exemplary knee braces. While only knee braces are illustrated for simplicity, it is understood that similar features and concepts can be applied to elbow braces and other limb braces that accommodate a joint of the user.

Also, while the braces illustrated herein are shown in a generally upright orientation as they would be worn on the leg of a user, it is understood that other braces may have other configurations and orientations. Similarly, any references to "upper" and "lower", or "front" and "rear", or "left" and "right" or other such relation terms are used for convenience and with respect to the illustration of a given embodiment shown. For convenience, the upper portion of the illustrated knee braces is the portion that is intended to be positioned about the upper leg or thigh of the user, and would be physically above the user's knee when a user is standing. Conversely, the lower portion of the illustrated knee braces is the portion that is intended to be positioned about the lower leg or calf/shin of the user, and would be physically below the user's knee when a user is standing. However, these are not limiting terms and features that are described as being on an "upper" frame as illustrated could alternatively be provided on a "lower" frame in other embodiments of the brace, and vice versa.

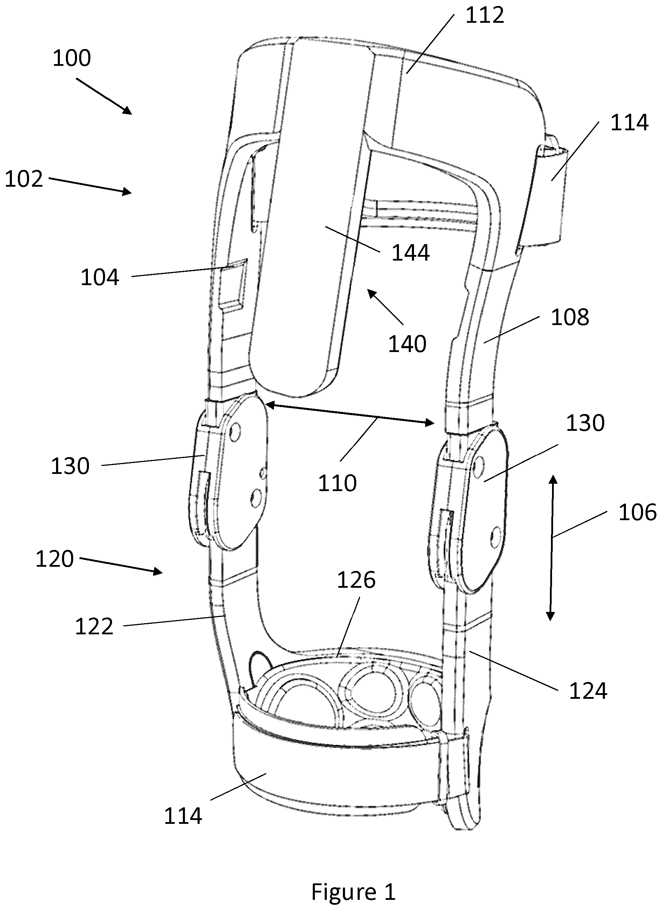

Referring to Figures 1-3, one exemplary embodiment of a brace 100 that is configured as a knee brace for a human user. This brace 100 includes an upper frame 102 that is designed to engage the upper leg or thigh of the user. The upper frame 102 includes a first arm that extends in a generally longitudinal direction (see arrow 106) and an opposing second arm 108 that also extends longitudinally and is offset from first arm 104 in a lateral direction (see arrow 110). The upper arms 104 and 108 may be generally symmetrical or may have different shapes to help accommodate the specific shape of a user's leg. Similarly, the upper arms 104 and 108 may be substantially linear, or may be curved and have a least some non-linear sections.

To help keep the arms 104 and 108 in their desired orientation, and to help engage and bear against the leg of the user, the upper frame 102 also preferably includes at least one structural cross-member that extends laterally between the arms 104 and 108. The cross-member is preferably shaped to be at least somewhat complimentary in shape to the thigh of a user, and can be formed from a relatively stiff, strong material as it may carry some loads while the brace 100 is in use. More than one cross-member may be provided in some embodiments of a knee brace, or a given cross-member may include cut-outs, gaps, or other such features that may affect the stiffness, weight or aesthetic features of the brace. Optionally, the cross-member on the upper frame can be provided on the anterior or front side of the brace 100, such that when the brace 100 is in use the cross-member overlies and bears against the front of the thigh/ quadriceps of the user. Optionally, cross-member(s) may only be provided on the anterior side of the upper frame, and the posterior or rear side of the upper frame may be free from structural cross-members (it may however include a strap or other suitable type of fastener). Alternatively, the upper frame 102 may be configured so that its cross-member(s) is located on the rear or posterior side of the frame so as to overlie and bear against the rear of the users thigh/ hamstrings when the brace 100 is in use. Optionally, cross-member(s) may only be provided on the posterior side of the upper frame, and the opposing anterior or front side of the upper frame may be free from structural cross-members (it may however include a strap or other suitable type of fastener).

In the illustrated example, the upper frame 102 includes a cross-member 112 that extends generally laterally between and joins the upper ends of the arms 104 and 108. The cross-member 112 is slightly convex and is curved to better conform to the expected shape of the user's leg. While shown as joining the upper ends of the arms 104 and 108 in this example, it is possible in other embodiments that the cross-member 112 may be located at a different location along the length of the arms 104 and 108, such as being longitudinally inboard from their upper ends.

Preferably, the upper frame 102 can also include at least one fastener that can be used to secure the upper frame 102 to the leg of the user, such that the upper frame 102 will tend to be joined to and move with the upper leg of the user. The fastener may include any suitable apparatus, including belts, straps, clips, buckles, hook and loop closures, snaps and the like, and preferably is releasable by the user to help facilitate putting the brace 100 on an off. In the illustrated example, a fastening strap 114, formed from a flexible fabric web, is connected to the upper end of the upper frame 102 and can be secured and released using an integrated hook and loop closure assembly. Other strap designs or fasteners could also be used.

In this example, the brace 100 also includes a lower frame 120 that is movably connected to the upper frame 102 and is designed to engage the lower leg or calf/shin of the user. The lower frame 120 includes a first arm 122 that extends in a generally longitudinal direction and an opposing second arm 124 that also extends longitudinally and is offset from first arm 122 in a lateral direction. The lower arms 122 and 124 are, in this example, generally laterally aligned with their corresponding upper arm 104 and 108. The lower arms 122 and 124 may be generally symmetrical or may have different shapes to help accommodate the specific shape of a user's leg. Similarly, the lower arms 122 and 124 may be substantially linear, or may be curved and have a least some non-linear sections.

To help keep the lower arms 122 and 124 in their desired orientation, and to help engage and bear against the leg of the user, the lower frame 120 also preferably includes at least one structural cross-member that extends laterally between the lower arms 122 and 124. The cross-member is preferably shaped to be at least somewhat complimentary in shape to the lower leg of the user, and can be formed from a relatively stiff, strong material as it may carry some loads while the brace 100 is in use. The lower cross-member may have a similar configuration as the upper cross-member 112, or may have a different shape and configuration. More than one lower cross-members may be provided in some embodiments of a knee brace, or a given cross-member may include cut-outs, gaps, or other such features that may affect the stiffness, weight or aesthetic features of the brace. Optionally, the lower cross-member on the lower frame 120 can be provided on the anterior or front side of the brace 100, such that when the brace 100 is in use the cross-member overlies and bears against the front of the shin of the user. Optionally, cross-member(s) may only be provided on the anterior side of the lower frame 120, and the posterior or rear side of the lower frame 120 may be free from structural cross-members (it may however include a strap or other suitable type of fastener). Alternatively, the lower frame 120 may be configured so that its lower cross-member(s) is located on the rear or posterior side of the frame 120 so as to overlie and bear against the rear of the users leg/ calf when the brace 100 is in use. Optionally, cross-member(s) may only be provided on the posterior side of the lower frame 120, and the opposing anterior or front side of the lower frame 120 may be free from structural cross-members (it may however include a strap or other suitable type of fastener).

Optionally, the brace 100 may be configured so that the upper and lower cross-members are provided on the same side of the brace 100, such that both cross-members are located on the anterior or front side of the brace and the rear side is free from cross-members, or such that both cross-members are located on the posterior or rear side of the brace and the front side is free from cross-members. Locating all of the cross-members on the same side of the brace may help simplify attaching and detaching the brace 100 from the user's leg. Alternatively, the brace 100 may be configured, as shown in this example, with the upper and lower cross-members on opposing sides of the brace 100. Specifically, positioning at least the lower cross-member on the posterior side of the lower frame 120 may help with the transfer of force between the brace 100 and the user's leg when the brace 100 is flexed. This may be preferably in some embodiments, but in other embodiments the lower cross-member may be on the front side and the upper cross-member may be on the rear side.

In the illustrated example, the lower frame 120 includes a cross-member 126 that extends generally laterally between and joins the lower ends of the lower arms 122 and 124. The cross-member 126 is slightly convex and is curved to better conform to the expected shape of the user's leg. While show as joining the upper ends of the lower arms 122 and 124 in this example, it is possible in other embodiments that the cross-member 112 may be located at a different location along the length of the lower arms 122 and 124, such as being longitudinally inboard from their lower ends.

Preferably, the lower frame 120 can also include at least one fastener that can be used to secure the lower frame 120 to the leg of the user, such that the lower frame 120 will tend to be joined to and move with the lower leg of the user. The fastener may be include suitable apparatus, including belts, straps, clips, buckles, hook and loop closures, snaps and the like, and preferably is releasable by the user to help facilitate putting the brace 100 on an off. In the illustrated example, another fastening strap 114 is connected to the lower end of the lower frame 120 and can be secured and released using an integrated hook and loop closure assembly. Other strap designs or fasteners could also be used.

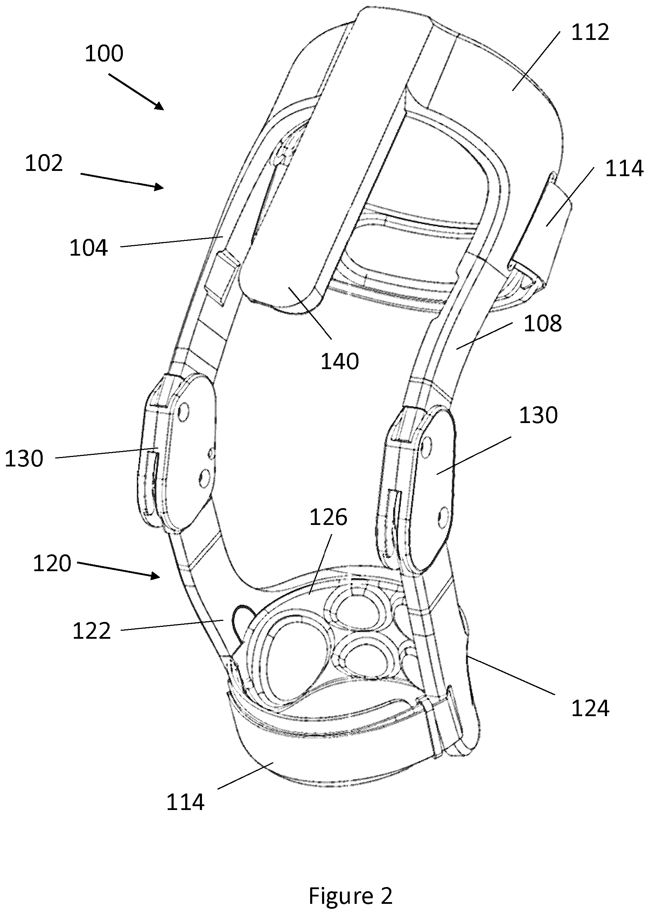

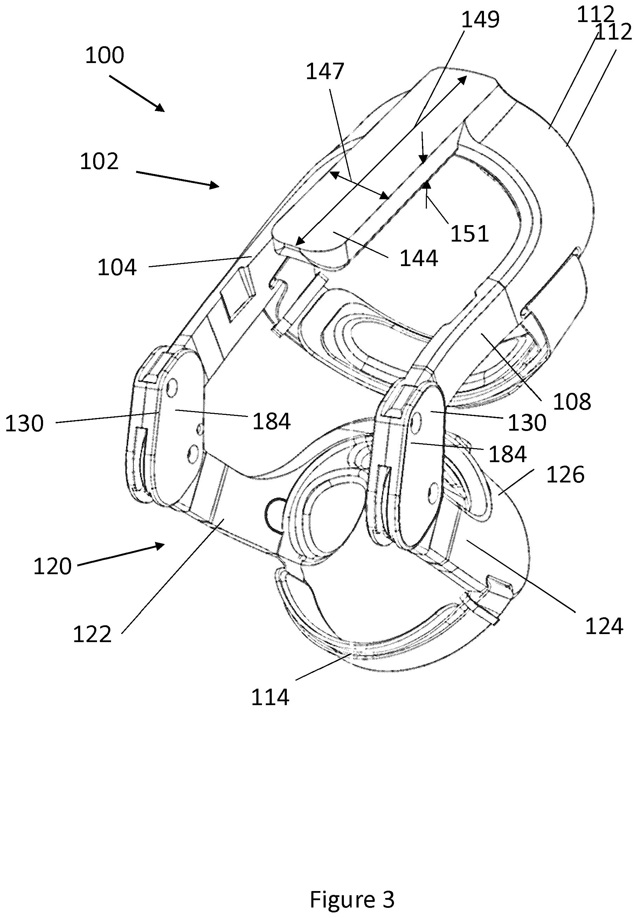

To allow the brace 100 to move with the leg of the user, the brace 100 includes a pair of hinges 130 that pivotally connect the upper frame 102 and lower frame 120 so that the brace 100 can be moved from an extended or straight position (Figure 1) to a flexed position (Figure 3), and can pass through one or more intermediate positions (Figure 2) along the way. The ultimate range of motion of the brace 100, between the extended and flexed positions, can be limited by including flexure stops or similar features or otherwise limiting the range of motion of the hinges 130. In addition to flexure stops, or as an alternative to flexure stops, the braces may also include suitable extension stops or similar features that can be used to limit the extension of the brace and optionally may keep the brace from reaching the fully extended position of Figure 1. While the embodiments described herein are configured so that there is little spring force acting when the brace is extended (Figure 1) and that flexing away from the extended position loads the spring, it is also possible that the neutral or low force position of the brace could be something other than the extended position. For example, the braces could be configured so that there is little to no spring force acting when the braces are flexed, and extending the brace tends to load the spring so that the restorative force moves the brace toward the flexed position. It is also possible for the braces and the energy recover systems described to be configured so that the "neutral" position for the hinge and brace is another position, such as an intermediary position, such as when the brace is flexed about 45 degrees. In this arrangement, moving the brace toward the extended or flexed positions could load the spring, and the brace generated a restorative force would urge the brace back to is partially flexed, neutral position. Variations of this nature may help facilitate the used of the braces rehab protocols and other specialized circumstances. Preferably, the hinges 130 on both sides of the brace 100 can have the same, or at least substantially the same construction and function, as shown in this example. Alternatively, different types of hinges may be used on opposite sides of the brace 100.

In this example, the hinges 130 are configured as polycentric hinges and can allow the upper and lower frames 102 and 120 to pivot relative to each other about a pivot axis 132 that can be aligned with the joint of the user when the brace 100 is in use.

The brace 100 is also configured to store energy when the brace 100 is flexed (e.g. move from the position of Figure 1 toward the position of Figure 3) and then to use that stored energy to apply a restorative force on the brace 100 that biases/urges the brace 100 to return from its flexed position to its extended position (e.g. move from the position of Figure 3 toward the position of Figure 1). The ability to absorb and store energy as the brace 100 is flexed can help reduce the amount of force that is carried by the user's limb/joint, and the restorative force can help assist the movement of the user's leg toward the extended position. To provide this energy storage and restorative force capability the brace 100 includes an energy storage assembly that contains at least one spring member that is arranged so that the spring member is charged (i.e. loaded) when the brace 100 is flexed, and so that the resistive, spring forces generated by the spring member in its charged state can serve as the restorative forces that urge the brace 100 toward its extended position.

As described herein, the spring member in a given embodiment of the brace may include a tension spring member, a compression spring member or a combination of different spring member types. Also, each spring member may include one, two, three or more separate springs or spring components that are arranged to work together to store energy when the brace is flexed and that can contribute to the restorative force that urges the brace toward the extended position. For example, a given spring member may include two or more tension springs (such as coil springs, elastics or elastomeric members and the like) arranged in parallel or in series with each other to provide the desired level of spring resistance and feedback. Alternatively, a spring member may include two or more hydraulic compression springs (with respective cylinders and pistons).

While some conventional braces can include some type of spring or energy storage mechanism that is incorporated into their hinges, the present brace 100 has been advantageously designed such that the energy storage assembly, and specifically at least the spring member that is used to store the energy in the system is remote from the hinges 130, and preferably is not mounted to or incorporated the hinges 130 or the upper arms 102 and 108 or lower arms 122 and 124. Locating the spring member (e.g. the energy storage device) at a location that is not in the hinges 130 or arms 102, 108, 122 and 124 may help reduce the overall size or bulk of the brace 100 and may allow the hinges 130 and arms 102, 108, 122 and 124 to be relatively smaller and thinner and/or differently shaped than arms of braces where the energy storage member is contained in the hinge or arm. However, with the spring member being remote from the hinge, a new system is required for transmitting the bending motion of the brace 100 (about hinges 130) into a force that can act on the remote spring member and load/charge the spring member. To provide this connection the inventors have developed a new system that utilizes an elongate and flexible tensioning member or cord that can pass thorough a corresponding cord path that is provided in the upper and/or lower frames 102 and 120 and extends from a location at or proximate the hinges 130 to the location of the energy storage assembly, and specifically the spring member. Examples of suitable spring members, cord paths and cord configurations are described herein, but other arrangements are also possible.

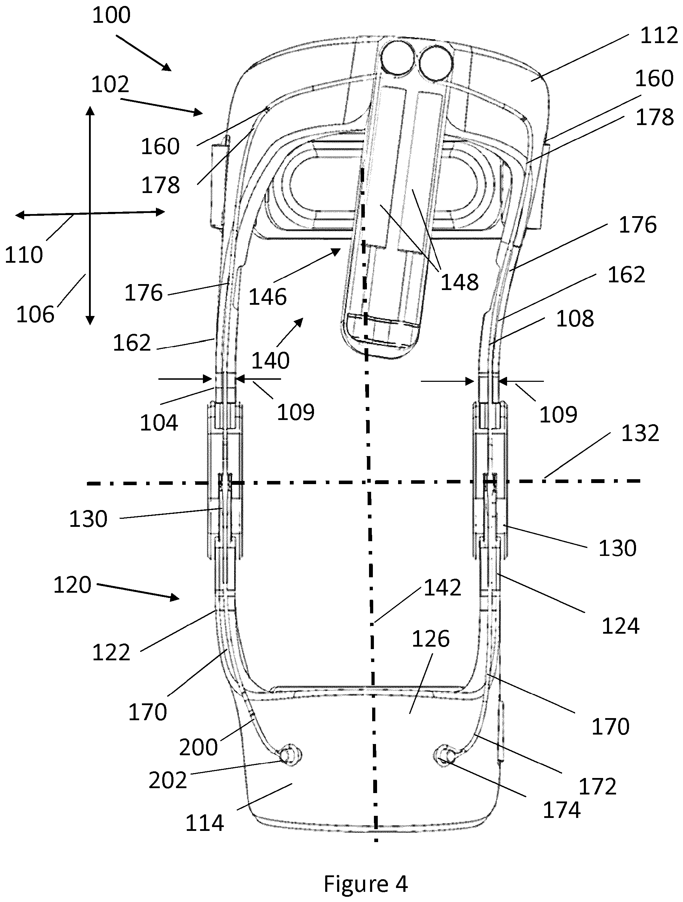

Referring to Figure 1, the brace 100 includes an energy storage assembly 140 that is mounted to, and supported by the upper cross-member 112 and is provided on the front side of the brace 100. In this location, the energy storage assembly 140 is longitudinally offset from the hinges and is located toward the top of the brace 100. This location is also laterally offset and inboard from the arms 104 and 108, and is preferably located toward the lateral centre of the brace 100, such that the spring member 146 is intersected by the central axis 142 (Figure 4) of the brace 100. While the energy storage assembly 140 is shown generally in the lateral centre of the brace 100 and toward the top edge, in other arrangements the energy storage assembly 140 may be offset laterally toward one side or the other, and need not be located at the top of the upper frame 102. In some alternative examples, the energy storage assembly 140 may be provided on the lower frame 120 and/or may be located on the rear side of the brace 100 rather than the front side. For example, if the upper cross-member 122 is located on the posterior side of the brace 100 then the energy storage assembly 140 may also be located on the outermost, posterior surface of the upper cross-member 112.

In this example, the energy storage assembly 140 includes a housing 144 (Figure1) which can optionally be integrally molded/formed with the upper cross-member 112 and/or other portions of the upper frame 102, or may be a separate component that is mounted to the frame 102. A spring member 146 (Figure 4) is positioned within the interior of the housing 144.

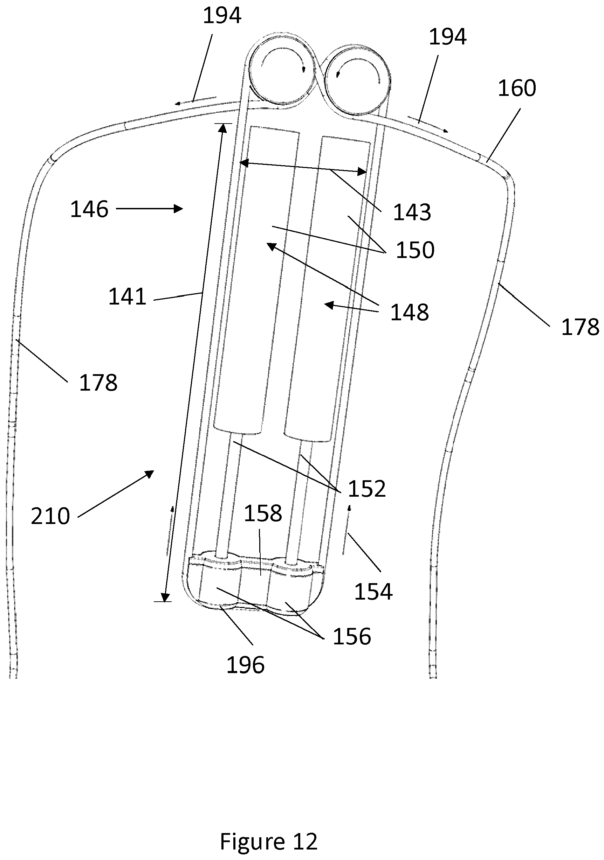

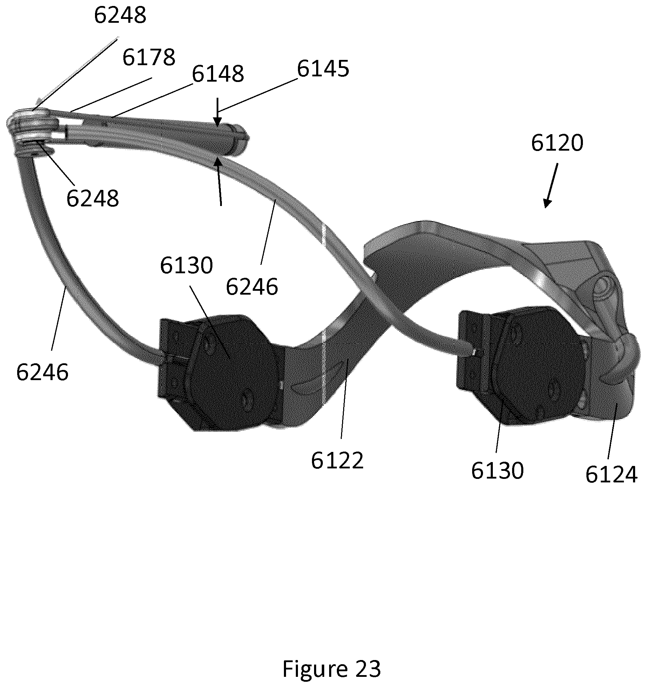

The spring member 146 may be any suitable spring or energy storage mechanism, and in this example includes two hydraulic compression springs 148 arranged in parallel with each other. Referring also to Figures 12-14, each compression spring 148 includes a respective cylinder 150 having a liquid and gas impermeable sidewall having a thickness that resists deformation under the maximum forces ordinarily encountered in the environment in which the spring 148 is used. The open end of each cylinder 150 (the lower end as illustrated in Figure 12) is closable with any suitable cap or cover member to enclose the interior of the cylinder 150. In this example, the spring member 146 has a spring length 141 (Figure 12), a spring width 143 and a spring thickness that is measured in the front/back direction and is generally into the page as illustrated in Figure 12, and is schematically represented by the thickness 6145 in Figure 23. The spring length 141, a spring width 143 and a spring thickness can be any suitable dimensions that can accommodate the desired spring mechanism. The housing 144 has an analogous housing length 149 (Figure 3), a housing width 147 and a housing thickness 151. By positioning the spring member 146 in the housing 144 at a location that is spaced from the arms 104 and 108, the arms can be constructed to have an arm width 109 in the lateral direction (Figure 4) that is less than at least one of the spring length, width and thickness, and preferably that is less than each of the spring length, width and thickness. This can help reduce the overall lateral size of the brace 100.

Each cylinder 150 slidably receives a respective piston having a piston rod 152 that is slidable through capped end of the cylinder 150 along a translation direction 154. The piston rods 152 can be formed from any suitable material, including for example hardened steel or another suitably durable material capable of being formed to high-precision tolerances. The piston rod 152 has a free or outer end 156 that can be engaged by other portions of the energy storage mechanism when the spring 148 is in use. The outer ends 156 of the piston rods 152 may be separate from each other and may be independently movable or, as shown in this example, may be connected with a bridge 158 so that the piston rods 152 are linked together.

The interior of the cylinder 150 has a liquid containment region that is preferably filled with a liquid, for example a silicone-based liquid or other suitable hydraulic fluid having the desired compressibility characteristics. Thus, when the springs 148 are unloaded and are in their rest or unloaded position as shown in Figure 12 (which corresponds to the situation when the brace 100 is extended as shown in Figure 1) the liquid fills the liquid containment space and is in a substantially uncompressed condition. In addition to the spring member 146, the brace 150 also includes a tensioning mechanism that is operable to transmit the movement of the brace 100 to the spring member 146 so that flexing the brace 100 can load the springs 148.

In this example, the brace 100 includes a flexible and preferably at least substantially inelastic tensioning cord 160 that runs through a corresponding cord path 162 within portions of the brace 100. The cord path 162 can include a plurality of passages or conduits that are formed in the upper and lower frames 102 and 120, or alternatively the cord 160 may be routed on the outer surfaces of the frames 102 and 120 or other suitable locations.

Referring to Figure 4 and also to Figures 12-14 which show the springs 148 and a portion of the cord 160 in isolation for clarity, the tensioning cord 160 is preferably configured so that it has a first anchor section 170 that extends in a portion of the cord path provided in the lower frame and that is secured so as to be generally fixed relative to the lower frame 120 when the brace 100 is in use. In the example of Figure 4, the first anchor section 170 includes a first end of the cord 172 that is attached to a respective anchor point 174 on the lower frame 120. The portions of the cord path 162 that contain segments of the cord 160 that do not translate relative to the frame 120 when the brace 100 is in use can be referred to as static path portions.

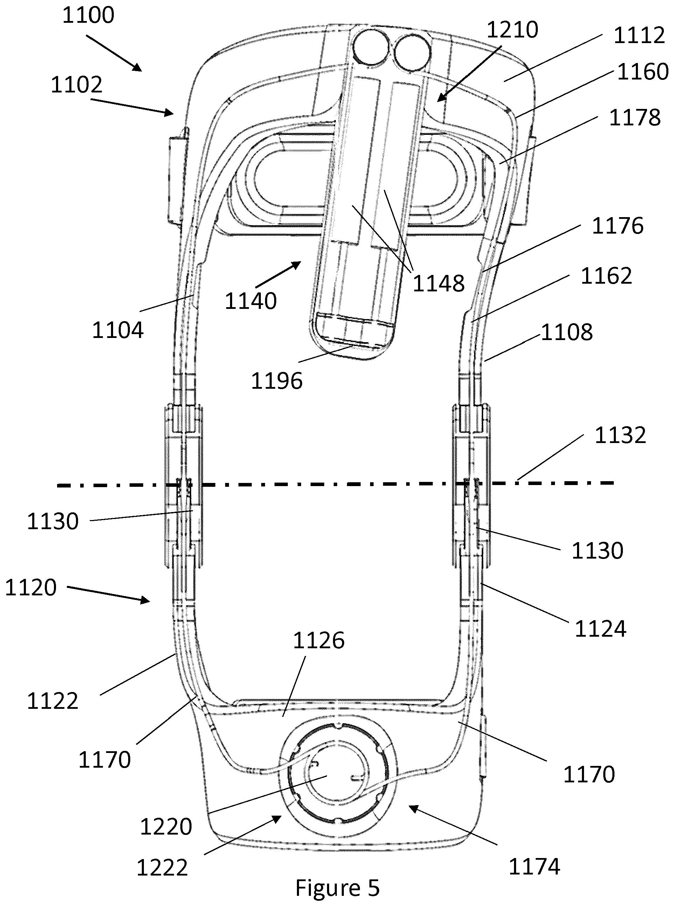

The cord 160 then continues along the cord path and extends through the hinge 130 connecting the upper and lower arms 108 and 124. The cord 160 then continues upwardly and travels thorough an upper portion of the cord path 162 that can allow a corresponding segment of the cord 160 to translate while the brace 100 is in use. This portion of the cord path 162 can be referred to as an energized cord path 176 and extends, in this example, form the hinge 130 to the energy storage assembly 140. The portion or segment 178 of the cord 160 within this energized cord path (e.g. extending form the hinge 130 to at least the energy storage assembly 140 when the brace 100 is extended) can translate within the energized cord path 176 when the brace 100 is flexed and extended. Preferably, at least some of the cord path can include channels that extend through the interior of the frames 102 and 120. The channels are preferably internal the frames 102 and 120 to help reduce the size of the brace 100 and/or to help reduce the likelihood of the cord 160 being exposed or damaged. Optionally, at least some portions of the cord path can be integrally formed with the upper and lower frames 102 and 120. This may help simply manufacturing of the brace 100. For example, in the embodiments of Figures 4 and 5,

To engage and apply a tension force on the cord 160 when the brace 100 is flexed, the hinges 130 can include an extension member, such as cam, lode or other shaped feature, that has a peripheral surface that is sized and configured to bear against the tensioning cord 160, and to alter the path of the cord 160 when the brace 100 is flexed.

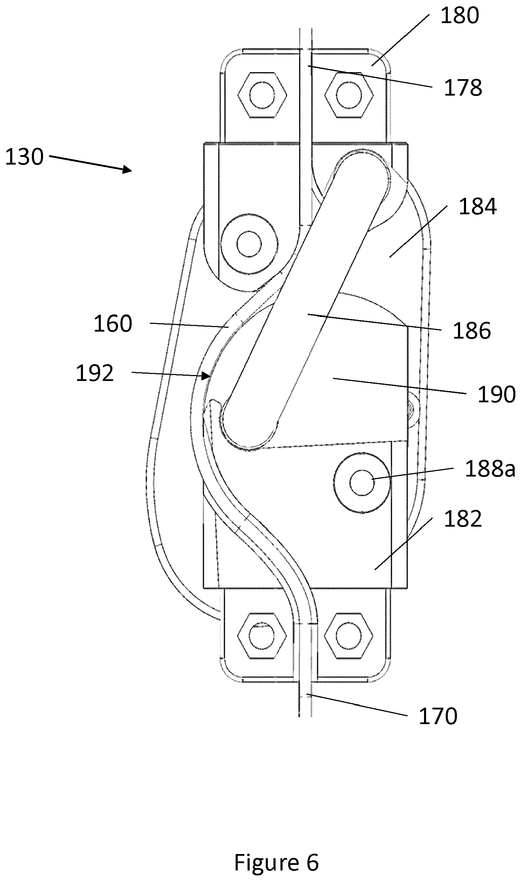

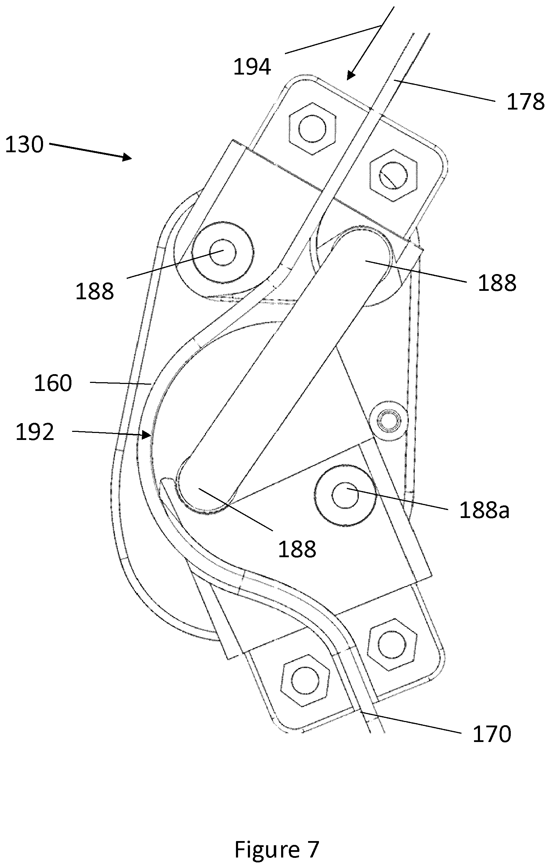

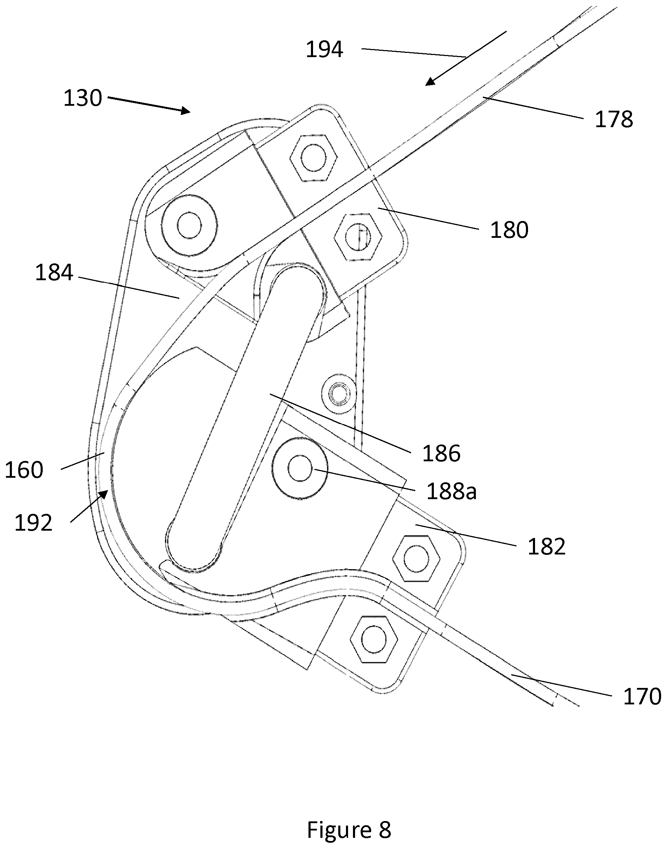

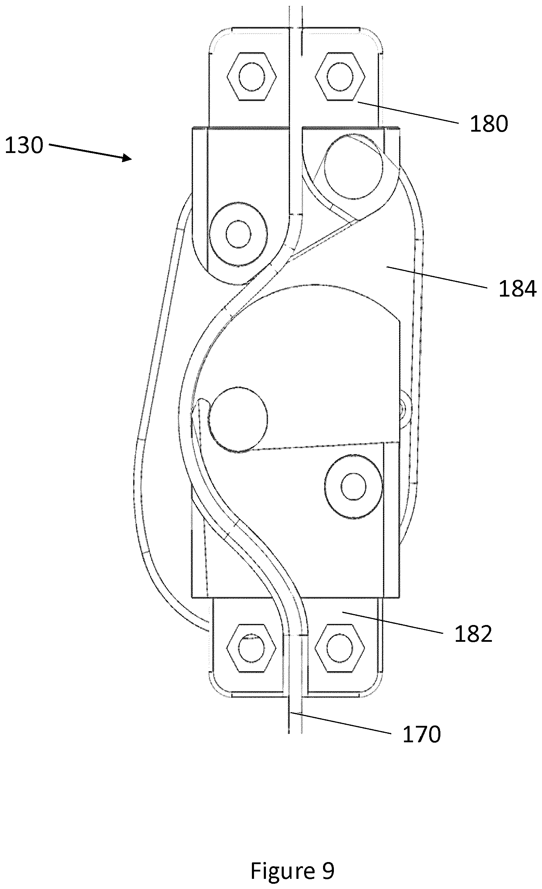

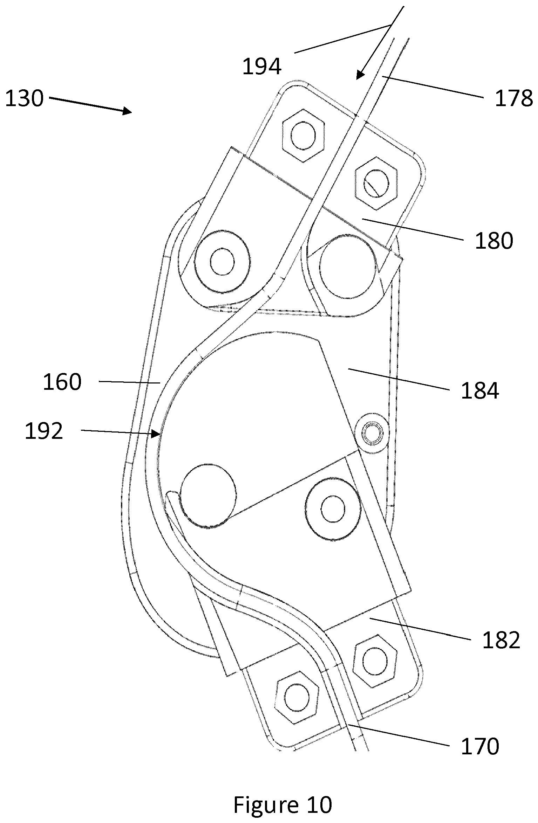

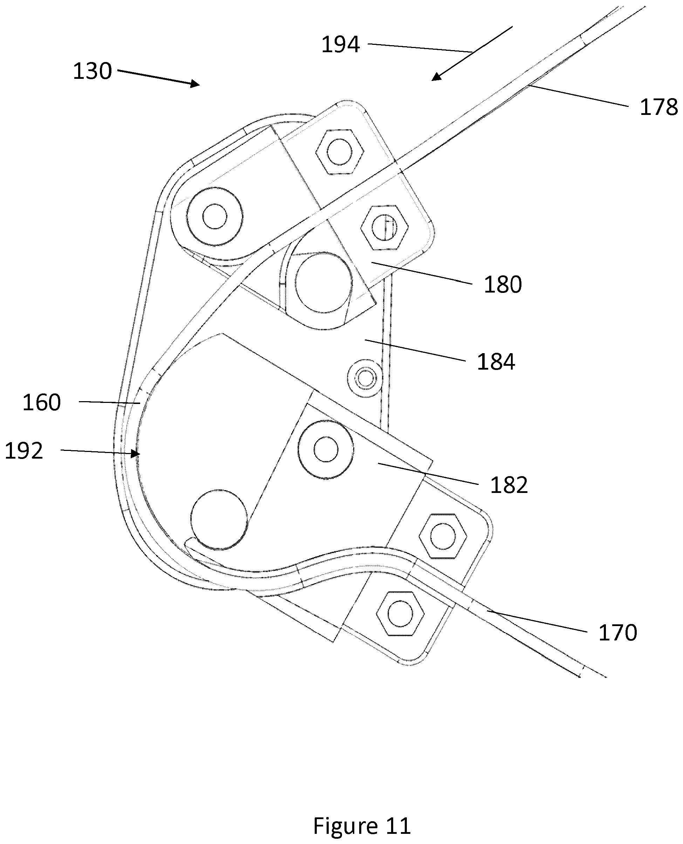

Referring also to Figures 6-11, one example of a suitable hinge design is illustrated, with portions of the cap/housing of the hinge 130 removed to more clearly show the interior features of the hinge 130. In this example, the hinge 130 includes an upper strut 180 that can be connected to an arm of the upper frame 102 and a lower strut 182 that can be connected to an arm of the lower frame 120. The struts 180 and 182 are pivotally connected to each other by a pair of outer links 184 and an inner link 186 via respective pivot joints 188 (Figure 8, for example). In this example, the lower strut 182 includes a cam portion 190 that has a curved, outer peripheral surface 192 that engages the tensioning cord 160. As the hinge 130 is flexed, from its extended position (Figure 6 - as corresponds to the brace in Figure 1) through its intermediate position (Figure 7 - as corresponds to the brace in Figure 2) to its flexed position (Figure 8 - as corresponds to the brace in Figure 3), the outer peripheral surface 192 deflects the cord 160 from its original position. Because the anchor section 170 of the cord 160 is fixed, this deflection of the portion of the cord 160 within the hinge 130 exerts a tension force (see arrow 194) on the energized portion of the cord 178. The specific shape and change in radius of the outer peripheral surface 192 can vary the amount of deflection, and therefore the resulting tension force 194, throughout the different range of motion of the hinge 130.

Preferably, in these examples, the tensioning cord 160 is at least substantially inelastic, and more preferably is inelastic to the extent that is not elastically expand when subjected to the expected loads it will be subjected to when the brace 100 is in use. This may be advantageous as the tensioning cord 160 is intended to be loaded under tension and to transmit that loading force to the springs 148 for storage. Elastic extension of the cord 160 could reduce the efficiency of this force transfer as energy would be absorbed by the extension of the cord 160 instead of via the springs 148.

An analogous configuration is included on the other side of the brace 100, as the hinge 130 connecting the upper and lower arms 104 and 122 can also include a segment 178 of the tensioning cord 160 that runs through a corresponding energized portion 176 of the cord path 162. A second anchor section 170 of the cord 160 extends through lower arm 122 and terminates a second end 200 that is secured to a second anchor point 202. In this arrangement, a single, continuous cord 160 extends from the first anchor 174, through the cord path 162 over the right hinge 130 along the upper arm 108, through the upper cross-member 112, engages the spring member 146, continues across the upper cross-member 112, extends through the upper arm 104, across the left hinge 130 and terminates at the second anchor 202. Using a continuous cord 160 in this manner can help provide generally equal tensioning and restorative forces on both sides of the brace 100.

To engage the spring member 146, the cord 160 may be connected to the spring member 146 in a variety of different ways, depending on the configuration of a given spring member and other portions of the brace. Referring also to Figures 12-14, in this example of the brace 100 that cord 160 includes a spring portion 196 that is connected to the ends 156 of the piston rods 152, or in this example to the bridge 158 that connects the ends 156. This spring portion 196 is connected such that it can exert the upward/inward compression force on the bridge 158, but preferably the spring portion 196 may be allowed to translate slightly relative to the bridge 158 when the brace 100 is in use. For example, by allowing the spring portion 196 to move, when the brace 100 is flexed, it can help accommodate for differences in tension between the energized cord segments 178 on the opposing sides of the energy storage assembly 140 it is handled with via a small translation of the cord 196 relative to the bridge 158. Such minor, rebalancing adjustments may happens automatically and instantaneously when the brace 100 is flexed, which can help provide at least substantially the same cord tension at each hinge 130 and thus, the same moment may be produced from each hinge 130. Alternatively, if the cord 160 were fixed relative to the spring member 146 a separate adjustment system could be provided on each side of the energy storage assembly 140 to dial in/ adjust the starting tension of each energized cord segment 178. This engagement can be press or friction fit between the bridge 158 and the cord 160, or may use fasteners, adhesives or other connecters to secure the spring portion 196 in place.

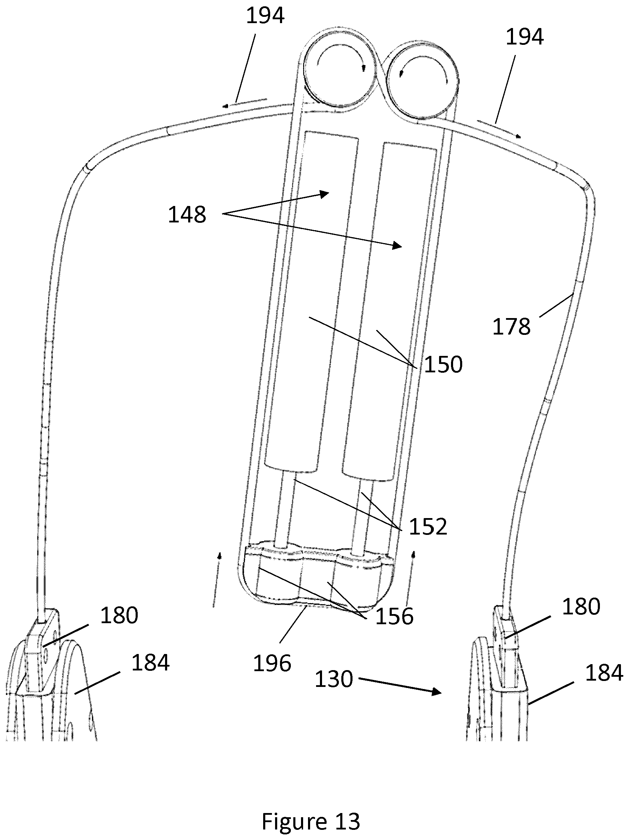

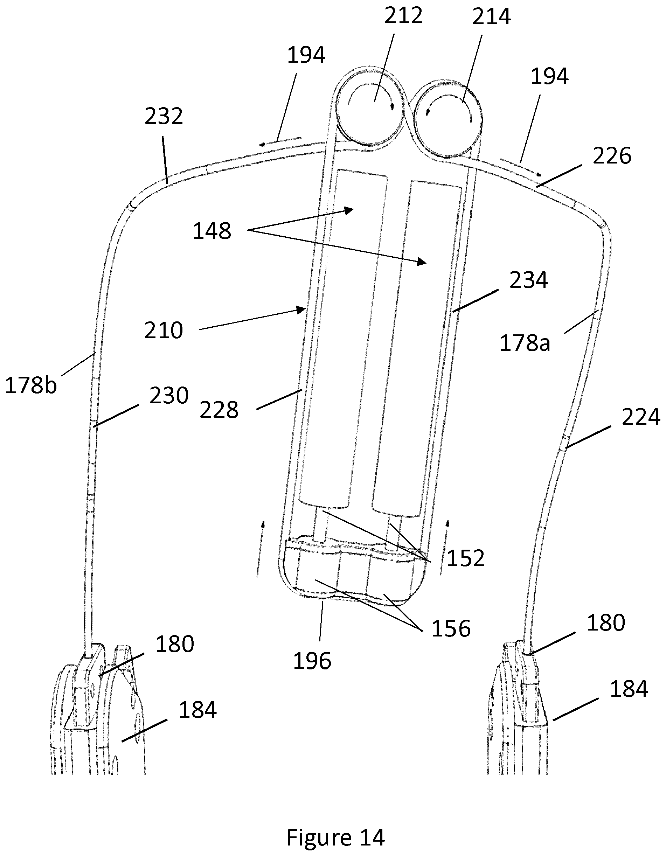

In addition to be connected via the bridge portion 196, the cord 160 in this example is advantageously arranged so that it forms a loop or bight region 210 (Figure 12) that is contained within the housing 144 (Figure 1) and generally encircles the spring member 146. In this configuration, the flexing of the brace 100 will exert tension forces 194 on both sides of the cord 160 which will tend to pull the cord 160 away from/ out of the housing 144. With the cord 160 looped to provide the bight, it can be seen in Figures 12-14 that pulling or translating the energized segments 178 of the cord 160 downwardly will draw other portions of the cord 160 away from the region of the spring member 146 and will constrict or shrink the bight region 210. Starting from the extended position in Figure 12 (which corresponds to the brace of Figure 1), the bight region 210 is at its largest and the piston rods 152 are largely exposed outside their respective cylinders 150. As both sides of the cord 160 are pulled by the cam surfaces 192 in the hinges 130 (Figure 13), the tension forces carried by the cord 160 urge the bight region 210 to constrict and exert a generally upward (or inward relative to the bight region 210) force bridge 158 that urges the piston rods 152 into the cylinders 150 thereby loading/charging the springs 148. When the brace 100 reaches its flexed position of Figure 3, the bight region 210 is further constricted as shown in Figure 14, and the piston rods 152 are driven further into the cylinders 150 until the springs 148 can be considered fully loaded/charged.

When the outside bending force is reduced from the brace 100, such as when a user starts to straighten their leg, the springs 148 will begin to unload and the piston rods 152 will be urged to translate out of the cylinders 150. That is, when the external force applied to the brace 100 (such as when a user bends their knee) is sufficient to overcome the spring/restorative force the brace can be moved away from its neutral position (which is the extended position in the illustrated embodiments, but may be the flexed position or an intermediary position as described in some other examples) thereby loading or charging the spring. When the external force is reduced, partially removed or lowered below the threshold level for a given brace design/calibration, the energy recovery system in the brace 100 acts in reverse and returns energy rather than continuing to store energy. While being used in-situ the extemal force that bends the brace 100 is unlikely to removed completely, but references herein to the external forces being removed refer to the condition where the applied external force is less than the restorative force.

This outward translation of the piston rods 152 will cause a corresponding movement and restorative tension on the spring portion 196 of the cord 160, which will in turn exert a restorative tension on the charged segments 178 of the cord 160 - drawing them generally upwardly as illustrated in Figures 14 to 12. This pulling on the cord 160 will urge the hinges 130 to return to their extended position and will act to help bias/urge the brace 100 toward its extended position of Figure 1.

To help guide the movement of the cord 160, and optionally to help reduce friction as the cord 160 moves, the energy storage assembly 140 can include one or more cord guides or aligning features. These may include active guide devices like pulleys and bearings, or passive guiding devices like bosses, static guide surfaces and the like. In the present example, the energy storage assembly 140 includes a pair of guide pulleys 212 and 214 that are positioned along the cord path, and preferably are within the housing 144. To help form the desired bight region 210, in this example the charged cord segment 178a on the right side of the assembly as shown in Figure 14 (for example) laterally crosses the springs 148 and is wrapped over the left hand pulley 212 before travelling longitudinally along the left or far side of the springs 148 to reach the spring portion 196. In this configuration, the charged cord segment 178a includes an outboard or first longitudinally oriented section 224 extending through the upper arm 108, a generally laterally extending transverse transition section 226 extending through the upper cross-member 112 and then an inboard or second longitudinally oriented section 228 that is within the housing 144 of the energy storage assembly 140. When the brace 100 is flexed, the outboard section 224 translates in one direction (downwardly as illustrated), while the inboard longitudinal section 228 translates in the opposite direction (upwardly as illustrated in Figure 14). These directions of translation are reversed when the brace 100 moves from the flexed position to the extended position. The lateral section 226 moves generally laterally outwardly and then inwardly as the brace is flexed and extended, respectively.

The other side of the brace 100 has analogous features and operations. For example, the energized cord segment 178b has an outboard longitudinal segment 230 that extends with upper arm 104, a transverse or laterally extending segment 232 that extends through the upper cross-member 112 and is wrapped over the right hand pulley 214 (as illustrated). An inboard longitudinally extending segment 234 extends from the pulley 214 toward the spring portion 196. When the brace 100 is flexed, the outboard section 230 translates in one direction (downwardly as illustrated), while the inboard longitudinal section 234 translates in the opposite direction (upwardly as illustrated in Figure 14). These directions of translation are reversed when the brace 100 moves from the flexed position to the extended position. The lateral section 232 moves generally laterally outwardly and then inwardly as the brace is flexed and extended, respectively - and moves in the generally opposite direction as the opposing transverse segment 226.

Preferably, the cord guides that engage and route the cord, such as the pulleys 214 and 216 in this example, can be connected to the spring member 146 in a generally rigid manner, such that the reaction or grounding force acting on the spring member 146 can be provided by the portions of the cord 160 that are opposite the spring portion 196. For example, in the present embodiment the pulleys 212 and 214 can be connected to the cylinders 150 (either directly or via a suitable bracket or connector) such that the pulleys 212 and 214 can rotate about their respective axes, but that any the inward, constriction forces exerted by the cord 160 as the bight region 210 constricts can be transferred to the cylinders 150, and reaction forces exerted by the cylinders 150 can be transferred to the cord 160. In this arrangement, constricting the bight region 210 can exert generally inward forces on both the upper ends of the cylinders 150 and the ends 156 of the piston rods 152, and substantially all of the reaction and restorative forces exerted by the springs 148 are transferred to and borne by the tensioning cord 160. That is, the restorative or expansion forces exerted by the springs 148 will tend to enlarge the bight region 210 and will be balanced by the tension forces in the cord 160, rather than being grounded to the upper frame 102. This can allow the springs 148 to be compressed and to apply the subsequent restorative forces without applying substantial grounding forces on the frame 102, which in turn may allow the frame to be formed from a wider range of materials than some convention designs in which the frame 102 has to carry substantially the entire restorative spring force.

While shown in one, generally upright orientation in Figures 12-14, the tensioning cord may include analogous longitudinally oriented and transverse sections if the spring member is mounted to other locations on the brace, including if the mounted to a lower cross-member.

In this example, the cord path was designed to reduce the grounding force the brace frame is required to sustain, further increasing the feasibility of lower strength materials (i.e. the ability to use the 3D printed plastics as described herein). The effect of this cord path is to significantly reduce the resultant grounding load required to be carried by the upper brace frame. Wrapping the spring member 146 in the bight 210 of the cord 160 causes the tension force in the cord 160 to act in a direction which partially cancels out the grounding force of the compressed springs 148. Following the cord's path in this example, the cord 160 extends from the right hinge 130 (as illustrated in these drawings) and through the energize cord path with the right, upper arm 108 and through the cross-member 112 toward the energy storage apparatus 140. The cord 160 then wraps around a first groove in pulley 214 by a wrap angle of approximately 90 degrees, and continues to pulley 212. At pulley 212, the cord 160 is then routed around the pulley by a wrap angle of approximately 180 degrees around a first groove in the pulley 212. From pulley 212, the cord extends longitudinally along the side of the spring member 146 (e.g. see segment 210 in Figure 14) until it reaches the bridge 156. The spring portion 196 of the cord then wraps approximately 180 degrees around the bridge 156 and extends along the opposite side of the spring member 146 (see segment 234) where the cord 160 then is received in a second groove that is formed in pulley 214 (and is preferably parallel to and offset from the first groove in pulley 214). The cord 160 wraps around the second groove by a wrap angle that is approximately 180 degrees and continues to laterally engage a corresponding second groove in the pulley 212 (and is preferably parallel to and offset from the first groove in pulley 212). In this arrangement, the pair of first grooves in the pulleys 212 and 214 are generally aligned with each other (e.g. can be generally co-planar and/or aligned in axial direction of the pulley or the front/back direction as illustrated) and the second grooves in the pulleys 212 and 214 are preferably generally aligned with each other and axially offset (e.g. in the direction of an axis of rotation of the pulleys) from the pair of first grooves. Having engaged the pulley 212 for the second time, the cord 160 can then continue laterally across the cross-member 112, through the upper arm 104 and toward the opposing, left hinge 130.

In this arrangement, the cord 160 generally exerts an upward or longitudinally outward force (generally away from the spring member 146 as illustrated) in the first groove of pulley 214, a downward force in the first groove of pulley 212, then a downward or longitudinally inwardly force on the second groove of pulley 214 (generally toward the spring member 146 as illustrated) and an upward force in the second groove of pulley 212. By counter wrapping the cord 160 around each pulley twice as described herein, at least a portion, and preferably a significant portion of the forces acting on the pulleys 212 and 214, and specifically a force acting between the bearings supporting each pulley 212 and 214 and the shaft the bearing is mounted on can be reduced because the opposing upward and downward forces acting on each pulley 212 and 214 can at least partially offset each other or cancel each other out. This may also help reduce the reaction force between the shafts supporting the pulleys 212 and 214 and whatever structure houses or supports the shaft. This can facilitate the use of smaller bearings than would be required to support an uncompensated application of the same cord tension. This may also facilitate the forming parts of the shafts, bearings and pulleys out of plastic rather than requiring the relatively stronger and heavier metal or alloy composition.

In this example the cord 160 is provided as a continuous, integrally formed length extending between the ends 172 and 200, and anchors 174 and 202. Alternatively, the cord 160 need not be of integral, one piece construction. In some alternatives, the cord may be formed from two or more sections of cord that are joined together. The sections may be the same, so that the cord 160 has homogeneous properties along its length, or may be different. Optionally, the cord 160 may be provided in two segments, each extending from a respective anchor 174 or 202 to the bridge 158. The portions of the cord disposed at the bridge may be connected to each other, or alternatively each portion may be fastened to the bridge 158 - or to individual ends 156 of the piston rods 152.

Optionally, the hinges 130 used in the braces described herein may be of any suitable design that provides sufficient strength and that can guide movement of the brace between the flexed and extended positions. This can include pin or pivot joints with a single pivoting joint, polycentric hinge designs and other suitable structures. Preferably, the hinges are configured to operate a polycentric hinges which can help the brace better track the motion of the wearer's knee joint (or other joint as appropriate). More preferably, the hinges can include a guiding mechanism that helps guide the movement of a given hinge (and can help limit over extension) and optionally that can synchronize the motion of both hinges so that both arms of the frame will move in unison when the brace is flexed and the frame will stay relatively balanced.

For example, the hinges may include intermeshing gear teeth on the upper and lower portions of the hinge that can mesh with each other and serve to guide and limit the rotation of the frame, such as is illustrated in U.S. patent no. 10,070,983 Other suitable structures could include cogs, belts and pulleys that can help achieve the desired polycentric motion. However, it has been discovered that when a brace is configured to provide a relatively high restorative force the gear teeth, for example, are subjected to relatively high loads when the brace is in use. This can contribute to wear and eventual failure of the teeth, which can limit the functionality of the brace prematurely (e.g. before the other components of the brace, such as the frame and spring member, have failed).

Instead of hinges with intermeshing gear teeth, the hinges 130 in the embodiment of Figures 6-11 are configured to utilize a mechanical, four bar linkage mechanism to create the "polycentric" motion on the upper and lower brace frames 102 and 120. The brace 100 therefore continues to track the motion of the wearer's knee joint, but uses a mechanism that is better suited to carry the loads created by the brace's relatively high energy storing springs 148. In this example, the links of the mechanism are (1) the cam/lower strut 182 , (2) the outer link(s), the upper strut 180, and (4) the inner link 186. The four bar linkage is a crossed parallelogram type. In this configuration shown in Figures 6-11, and the cam 190 is integrated into the ground link/ lower strut 182. It should be noted the cam 190 could alternatively be integrated into any of the four links, including the upper strut 180 in other examples. One advantage of this hinge design is that it both utilizes the four bar design to create a "polycentric motion" which approximates that of the knee joint, and that the cam 190 that is integrated into the lower strut link 182 also acts on the tension cord, causing the deflection of the cord 160 that helps generate the restorative forces.

Reducing the stiffness requirements of the frame 102 can help allow the brace 100 to be made by modern digital manufacturing techniques such as additive manufacturing and/or 3D printing. This may be particularly useful in some examples because (1) the brace frames can be designed to be truly custom by building the frame shape off a 3D scan of a wearer's leg, and (2) the hollow passages that form the cord path can follow complex 3-dimensional paths in the frame 102 and 120 in a way that traditional manufacturing techniques such as injection molding or machining could not easily achieve.

Also, in the present example, configuring the system so that the cord 160 loops over an the guide (or pulley) which bears on the spring member 146 has the effect of halving the tension carried by the each side of the cord loop. By implementing two symmetric hinges in the brace, each hinge is made to carry ½ the cord tension as would generated by a single hinge of equal torque output. This symmetry allows components to be made of cost-effective materials such as injection molded plastics. In addition, this configuration imparts symmetric forces on the brace, meaning the frames can be made lighter and thinner, as they require much less bending stiffness in the transverse plane.

Optionally, instead of the cord 160 being configured with separate ends that are individually anchored to the lower frame 120, an alternative embodiment of the brace can be configured such that the connection point between the cord and the frame can be adjustable. This may allow the tension on the cord to be adjusted, for example by adjusting how the cord is anchored, while the brace is in a given position (such as either extended or flexed). This may allow the amount of restorative force that is generated by the energy recovery system to be adjusted. For example, if the tension in the cord is increased while the brace is in the extended position it may have the effect of partially pre-loading the spring member. When the brace is then flexed, the corresponding compression of the spring member will be relatively greater than if the brace had been flexed with an initially looser cord. Allowing the tension on the cord to be manually adjustable by a user may allow the user to adjust the cord tension and overall restorative force provided by the brace from time to time.

Referring to Figure 5, another example of a brace 1100 includes one example of a tensioning adjustment mechanism that allows a user to adjust tension in the cord, and thereby adjust the restorative force provided. The brace 1100 is similar to brace 100, and analogous features are illustrated using like reference characters indexed by 1000. In this example, the brace 1100 includes upper and lower frames 1102 and 1120, each having respective arms 1104, 1108. 1122 and 1124 and cross-members 1112 and 1126. The upper and lower frames 1102 and 1120 are connected by hinges 1130, and an energy storage assembly 1140 is mounted to the upper cross-member 1112. As described herein, a tensioning cord 1160 is provided within a corresponding cord path 1162, and is arranged to pass through both hinges 1130 and is looped around the springs 1148 to provide a bight region 1210 that can be tightened/ constricted to squeeze the springs 1148 when the brace 1100 is flexed.

In contrast to the embodiment of Figure 4, in this example the tensioning cord 1160 is one, generally continuous loop and does not have separate ends that are fixedly anchored to the lower frame 1120. Instead, the anchor sections 1170 of cord 1160 are connected to each other to complete the continuous, looping cord 1160. To secure the lower portion of the cord 1160 to the frame 1120, the cord 1160 is wrapped around the rotatable spool/spindle 1220 of a winding apparatus 1222. In this arrangement, turning the spindle 1220 in one direction can wind the cord 1160 around the spindle 1220, which has the effect of drawing portions of the cord 1160 downwardly toward the apparatus 1222, thereby increasing the tension along the entire length of the cord 1160. Depending on the tightness, winding the cord 1160 in this manner may constrict the bight region 1210 and pre-load the springs 1148. Turning the spindle 1220 in the opposite direction can unwind the cord 1160, thereby loosening the cord 1160 and reducing the tension in the cord 1160. Once the cord 1160 is in the desired position the user can lock or otherwise immobilize the winding apparatus 1222, which can fix the anchor sections 1170 in a set position relative to the lower frame 1120. With the winding apparatus 1222 locked, the anchor sections 1170 do not translate relative to the lower frame 1120, and the remaining portions of the cord 1160 can operate as described herein, so that flexing the brace 1100 loads the springs 1148.

The winding apparatus 1222 can be any suitable type of mechanism, including a cord winding ratcheting pawl system (such as a BOA^{®}).

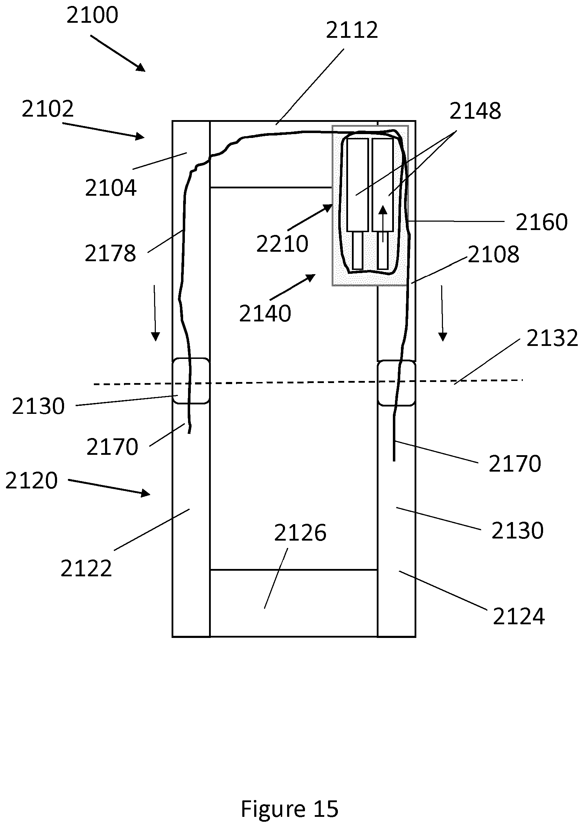

Referring to Figure 15, another example of a brace 2100 is schematically illustrated, and is configured so that the energy storage apparatus 2140 is offset toward one side of the brace 2100. The brace 2100 is similar to brace 100, and analogous features are illustrated using like reference characters indexed by 2000. In this example, the brace 2100 includes upper and lower frames 2102 and 2120, each having respective arms 2104, 2108, 2122 and 2124 and cross-members 2112 and 2126. The upper and lower frames 2102 and 2120 are connected by hinges 2130, and an energy storage assembly 2140 is mounted to the upper cross-member 2112, but is located laterally off-centre, toward one side of the brace 2100. As described herein, a tensioning cord 2160 is provided within a corresponding cord path, has anchor section 2170 that are fixed relative to the lower frame 2120, is arranged to pass through both hinges 2130 and is looped around the springs 2148 to provide a bight region 2210 that can be tightened/ constricted to squeeze the springs 2148 when the brace 2100 is flexed.

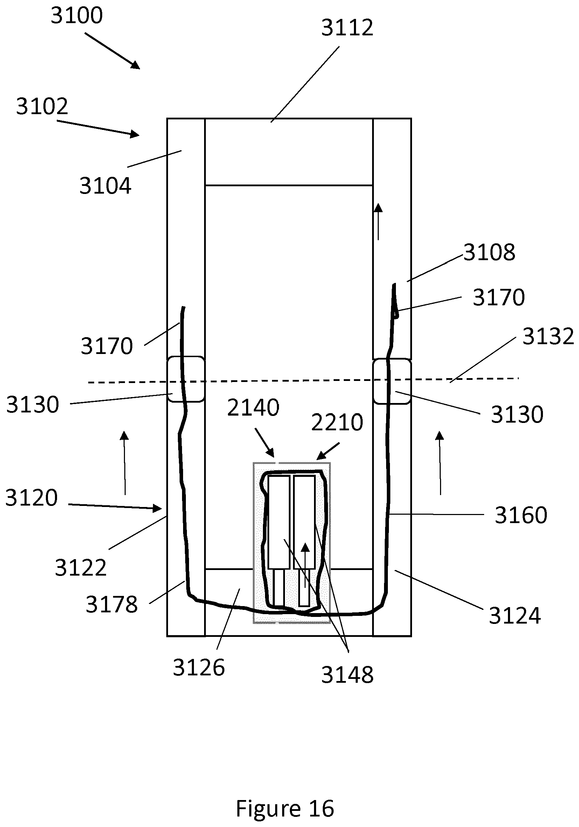

Referring to Figure 16, another example of a brace 3100 is schematically illustrated, and is configured so that the energy storage apparatus 3140 mounted to the lower frame 3120 instead of the upper frame 3102. The brace 3100 is similar to brace 100, and analogous features are illustrated using like reference characters indexed by 3000. In this example, the brace 3100 includes upper and lower frames 3102 and 3120, each having respective arms 3104, 3108, 3122 and 3124 and cross-members 3112 and 3126. The upper and lower frames 3102 and 3120 are connected by hinges 3130, and an energy storage assembly 3140 is mounted to the lower cross-member 3126. As described herein, a tensioning cord 3160 is provided within a corresponding cord path, has anchor sections 3170 that are fixed relative to the upper frame 3102 (rather than lower section 3120), is arranged to pass through both hinges 3130 and is looped around the springs 3148 to provide a bight region 3210 that can be tightened/ constricted to squeeze the springs 3148 when the brace 3100 is flexed.

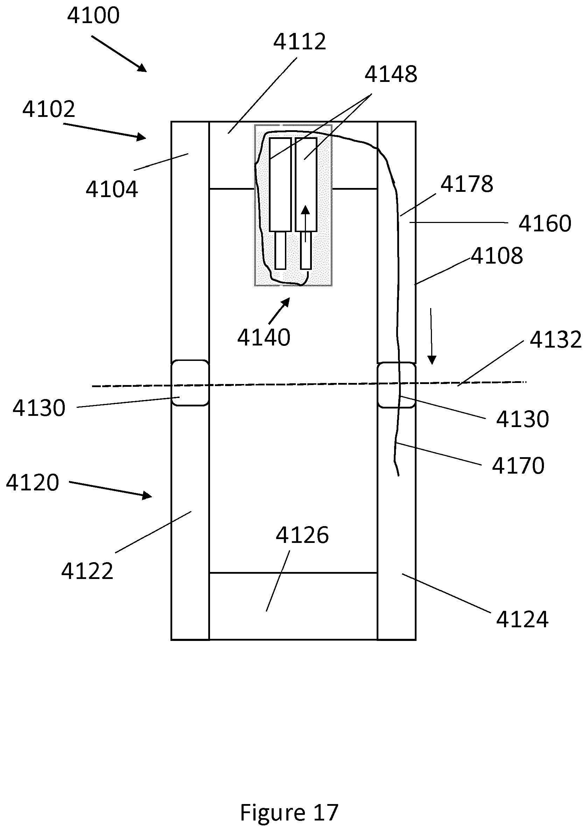

Referring to Figure 17, another example of a brace 4100 is schematically illustrated, and is configured so that it only includes a one-sided energy storage system, with a cord 4160 that is not configured as a continuous loop or member that extends through both sides of the brace 4100. Instead, the cord 4160 is only provided on one side, and extends from its anchor portion 4170 in lower arm 4124, across hinge 4130, through upper arm 4108 and into the energy storage apparatus 4140. Inside of the energy storage apparatus 4140, springs 4148 are configured to be compressed when tension is applied to the cord 4160, but the cord 4160 does not form a self-reinforcing bight portion. Instead, the springs 4148 can be grounded to the upper cross-member 4112. The brace 4100 is otherwise similar to brace 100, and analogous features are illustrated using like reference characters indexed by 4000. In this example, the brace 4100 includes upper and lower frames 4102 and 4120, each having respective arms 4104, 4108, 4122 and 4124 and cross-members 4112 and 4126. The upper and lower frames 4102 and 4120 are connected by hinges 4130, and an energy storage assembly 4140 is mounted to the upper cross-member 4115.

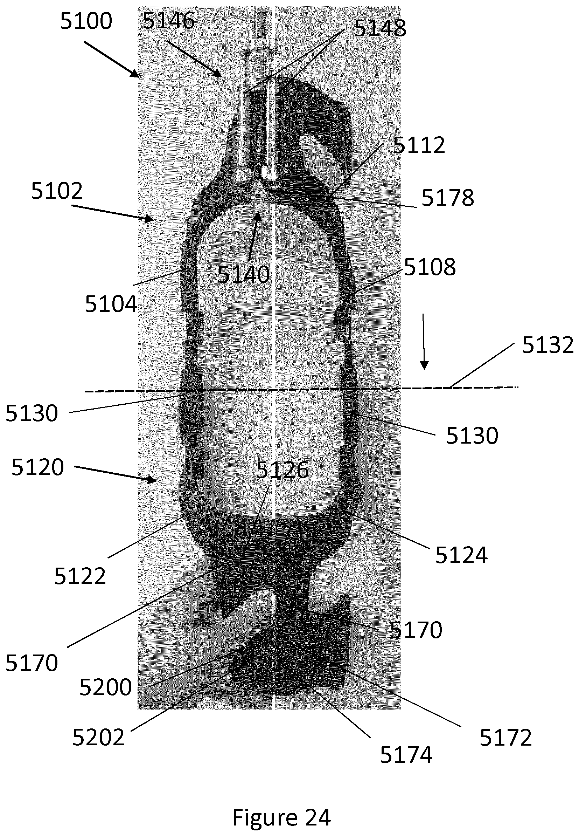

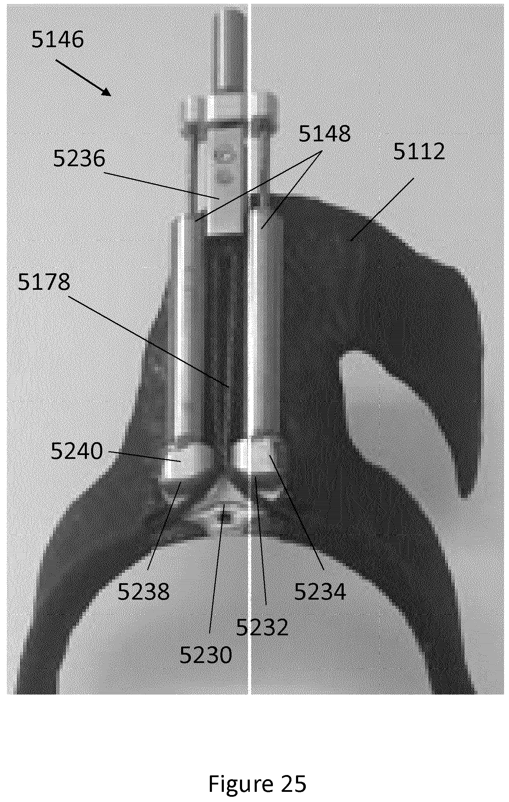

Referring to Figures 24 and 25, another embodiment of a brace 5100 is illustrated. The brace 5100 is generally similar to the brace 100, and like features are annotated using like reference characters indexed by 5000. In this example, the brace 5100 includes upper and lower frames 5102 and 5120, each having respective arms 5104, 5108, 5122 and 5124 and cross-members 5112 and 5126. The upper and lower frames 5102 and 5120 are connected by hinges 5130, and an energy storage assembly 5140 is mounted to the upper cross-member 5112. As described herein, a tensioning cord 5160 is provided within a corresponding cord path, has anchor sections 5170 next to cord ends 5172 and 5200 that are fixed relative to the lower frame 5120 at respective anchor points 5202 and 5174.

Instead of being configured so that the spring member 5146 is not directly grounded on the frame (as in the brace 100), in this example the spring member 5146 includes a set of two hydraulic springs 5148 that are installed on a rigid mount 5230 (Figure 25) on the cross-member 5112 of the upper frame 5102. Preferably, the mount 5230 is on the front or outer-facing surface of the cross-member 5112 as shown, but alternatively could be provided on the inner face or other suitable region. In this example, the brace's tensioning cord is made of a single length of braided rope and both of the ends 5172 and 5200 of the cord (e.g. the start and endpoint of the cord) are fixed to the anchoring portions 5174 and 5202 of the lower frame 5120. In this arrangement, a single cord 5160 is routed along the cord path through the hollow passages formed in the upper and lower frame 5102 and 5120s, as well as through both hinges 5130. This cord path is as follows: (i) bbeginning at a first grounding point 5174 in the lower frame 5120 and routing through hollow passages in the lower frame 5120 , (ii) the cord 5160 then exits the lower frame 5120, passes through the first hinge 5130 (including over a cam within the hinge) and then into a hollow passage within the upper frame 5102, (iii) the cord path moves through a hollow passage in the upper frame 5102, and then wraps 90° around a first bearing surface 5232 on the base of the spring mounting block 5234, up to and over the spring anchor block 5236, wrapping 180° over the anchor block 5236 then back down, wrapping 90° on a second bearing surface 5238 on the spring mounting block 5240 (see Figure 25). The cord 5160 then continues and is routed in a similar and symmetrical manner, through the opposite side of the upper frame 5102, the second hinge 5130 with a second cam, and finally back to the lower frame 5120 where the cord path terminates at a second grounding point 5202.

In this arrangement, when the brace 5100 is flexed, the cams in both hinges 5130 pull on the cord 5160 in the upper frame 5102. This motion of cam and cord action effectively causes the cord's path length through the upper frame 5102 to contract. This contraction of cord path length compresses the springs 5148 in the upper frame 5102. The tension transmitted to the cord 5160 by the spring compression force in the upper frame 5102 in turn creates a moment (torque), as this tension force acts at the radius of the respective cams of each hinge 5130. This moment is substantially equal to the cord tension multiplied by the radius distance of the cam. In effect, this arrangement of the 3-dimensional cord paths couples the motion of the cord 5160 in the hinge 5130 with the spring compression, and therefore energy storage, occurring in the upper frame 5102.

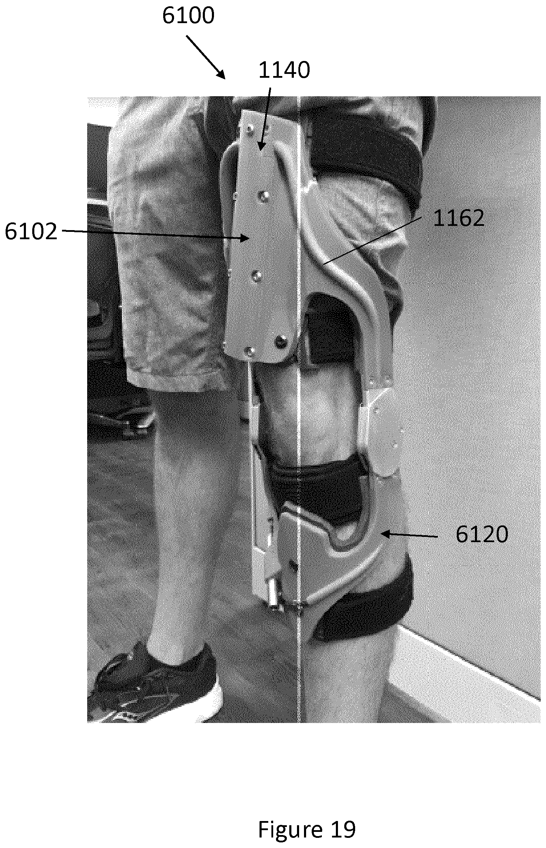

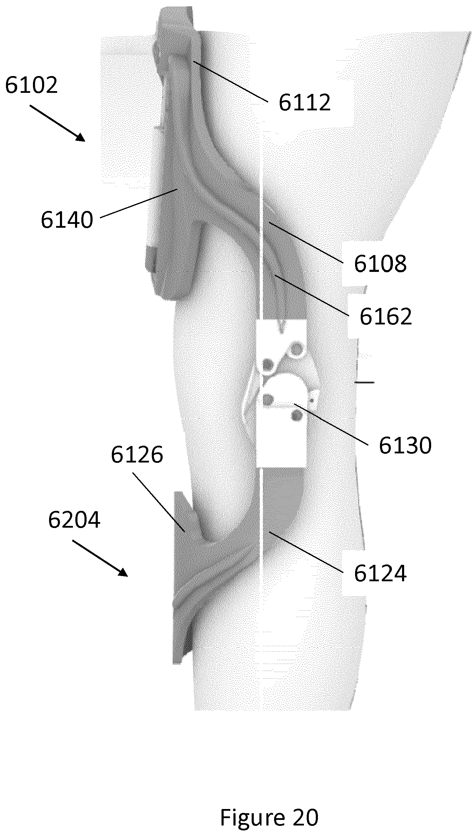

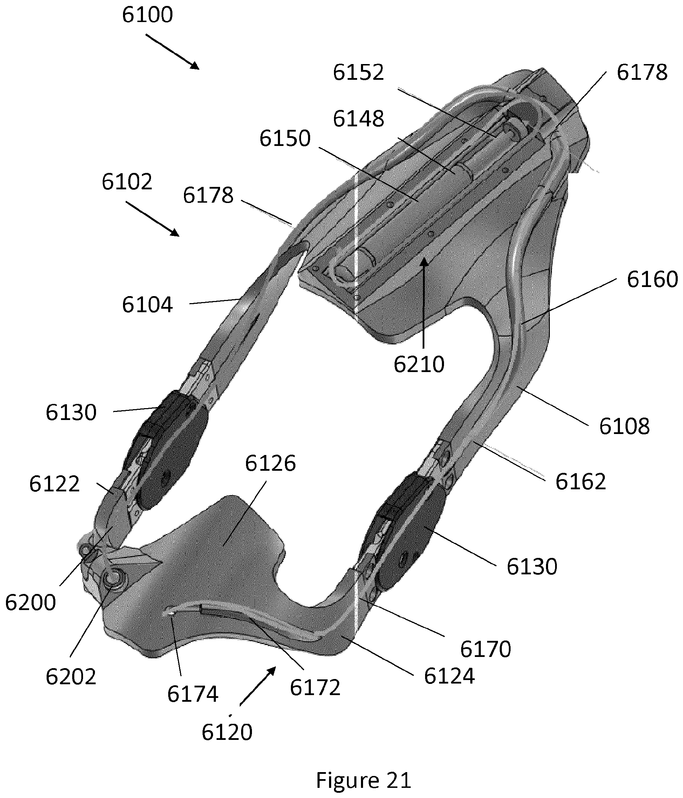

Optionally, instead of two hydraulic springs, a single hydraulic spring may be used. Referring to Figures 19-21, another example of a brace 6100 is illustrated. Brace 6100 is similar to brace 100, and analogous features are identified using like reference characters. In this example, the brace 6100 includes upper and lower frames 6102 and 6120, each having respective arms 6104, 6108, 6122 and 6124 and cross-members 6112 and 6126. The upper and lower frames 6102 and 6120 are connected by hinges 6130, and an energy storage assembly 6140 is mounted to the upper cross-member 6112. As described herein, a tensioning cord 6160 is provided within a corresponding cord path 6162, has anchor sections 6170 that are fixed relative to the lower frame 6120 at respective ends 6172 and 6200 via the anchor points 6202 and 6174, is arranged to pass through both hinges 6130 and is looped around the spring 6148 to provide a bight region 6210 that can be tightened/ constricted to squeeze the spring 6148 when the brace 6100 is flexed.

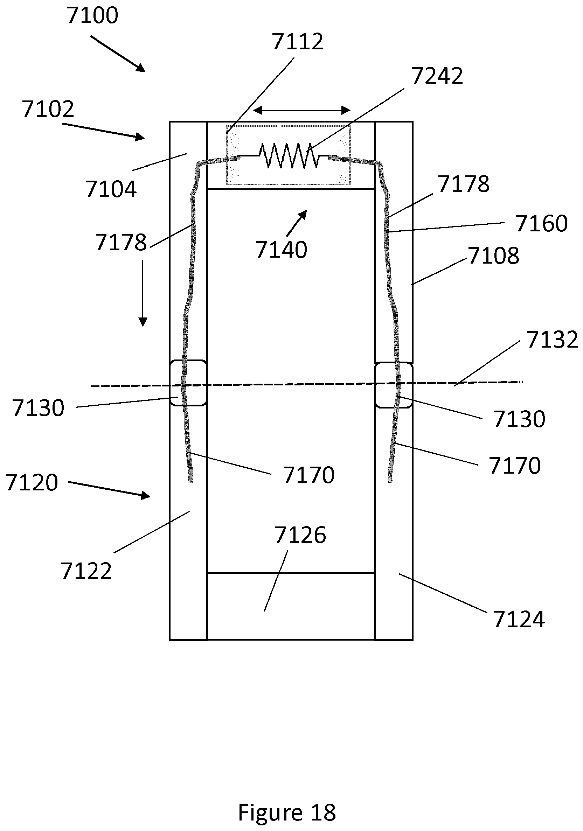

Optionally, instead of the hydraulic, compression springs illustrated in the previous embodiments, a brace may include one or more tension springs that can be spaced from the hinges, and can be engaged by the tensioning cord in an analogous manner. Referring to Figure 18, another example of a brace 7100 is schematically illustrated, and is configured so that the energy storage apparatus 7140 includes a tension spring member 7242, instead of a hydraulic compression spring. The brace 7100 is similar to brace 100, and analogous features are illustrated using like reference characters indexed by 7000. In this example, the brace 7100 includes upper and lower frames 7102 and 7120, each having respective arms 7104, 7108, 7122 and 7124 and cross-members 7112 and 7126. The upper and lower frames 7102 and 7120 are connected by hinges 7130, and an energy storage assembly 7140 is mounted to the upper cross-member 7112. As described herein, a tensioning cord 72160 is provided within a corresponding cord path, has anchor sections 7170 that are fixed relative to the lower frame 7120, is arranged to pass through both hinges 7130 but instead of being is looped around the springs to provide a bight region that can be tightened/ constricted to squeeze the springs, the energized sections 7170 of the cord 7160 are attached to opposite ends of the tension spring 7242. When the brace 7100 is flexed, the hinges 7130 displace the cord 7160 and exert a tension on the energized cord segments 7170, which applies a lateral tension to the spring 7242 thereby stretching and loading the spring 7242. When the outside flexing force is removed, the sprint 7242 will contract, thereby applying an opposite tension on the cord 7160 and urging the brace 7100 back to its extended position.

The cord described above, such as cord 160, herein may be formed from any material that is sufficiently flexible to follow the three-dimensional, circuitous cord path through the brace and that has a sufficient tensile strength to carry the expected tension loads without breaking. Some suitable examples of materials that can be used to provide the cord can include a multi-strand or braded rope made from natural or synthetic materials, a mono-filament rope, a wire, a single strand fibre/rope, a braded or multi-stranded wire, a composite structure including metal filaments wrapped in a non-metallic sheath, other composite structures and the like. The term cord as used herein is not limited to one specific material or configuration of flexible, tensioning carrying member.

One advantage of adopting the circuitous cord path, and optional wrapping of the cord around the spring member, is that this path arrangement may help reduce the stresses induced in the upper frame by the ground force of the compressed/loaded spring. However, this arrangement may lead to relatively increased energy loss to friction while the cords are pulled through the cord path when in motion (as compared to a generally linear cord path). Optionally, friction reducing coatings or elements can be used to help reduce the friction between the cords and the frames. This can include a relatively low friction sheath or coating that is applied to the cord or integrated into its design, a coating or lubricating layer that is applied to the interior surfaces of the cord path, the use of physical bearings, pulleys and other such features and/or inserting a tube or generally tube-like lining within the cord path that has a lower co-efficient of friction than the material that the frame is made from.

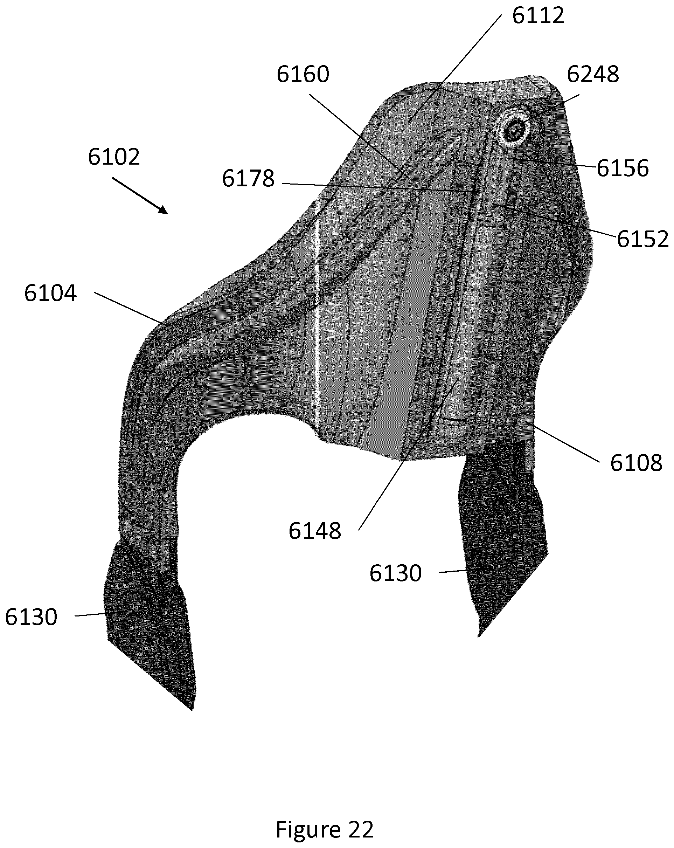

For example, referring to Figures 22 and 23, portions of the brace 6100 are shown. Figure 22 is a perspective view of the upper half of the brace 6100, while Figure 23 shows the brace 6100 with the upper frame 6102 hidden to reveal the interior portions (the cord path and friction reducing members) for clarity. In these examples, one embodiment of a friction reducing element can include relatively low friction plastic liner tubes 6246 that can be installed into, and line passages, in the frame 6102 (not shown in this Figure) that form the cord passages, thereby reducing the coefficient of friction against the moving cord. In this example, the liner tubes 6246 extend from the hinges 6130 to the energy storage assembly and terminate close to the spring 6148. The shape of these tubes 6246 can correspond to the shape of the cord path. These liner tubes 6246 can be formed from plastic (or another material) that is relatively smoother and optionally relatively softer than the material that is used to form the frames.

These liner tubes 6246 can optionally be used in both the upper and lower frame 6102 and 6120, or may be used in only one of the frames, and preferably are used in at least the upper frame 6102 where the movement of the cord 6160 within the passage is expected to be most pronounced.

Another example of a friction reducing member is the pair of counter-rotating pulleys 6248 and 6250 that are installed on the end 6156 of the piston rod 6152. These pulleys 6248 and 6250 can be used instead of a fixed or static cord guide surface and can allow for rolling contact at the two points in the cord's path 6160 where a 90° wrap angle occurs. This can help significantly reduce the friction at this point in the cord's 6160 motion. The pulleys 212 and 214 can have an analogous effect.

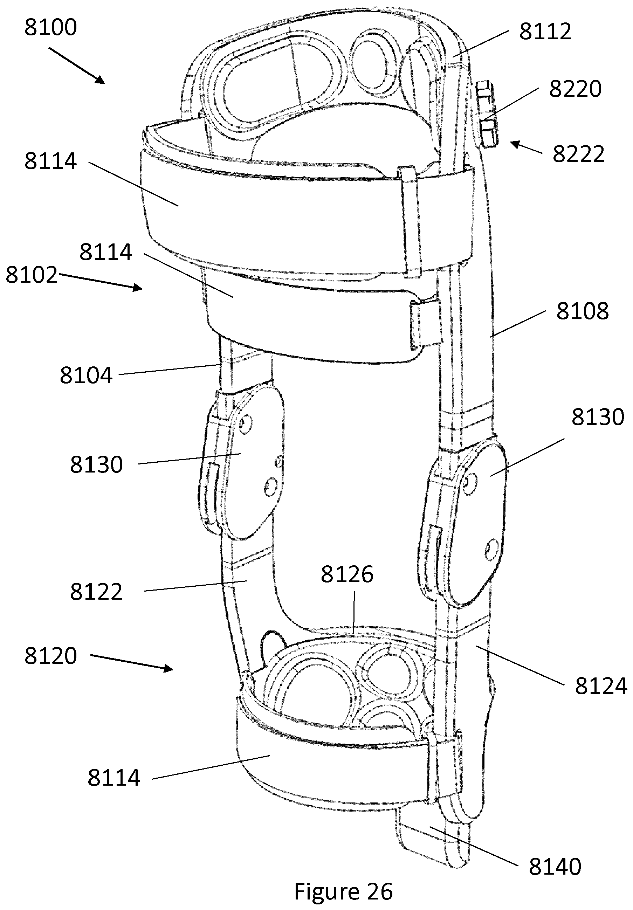

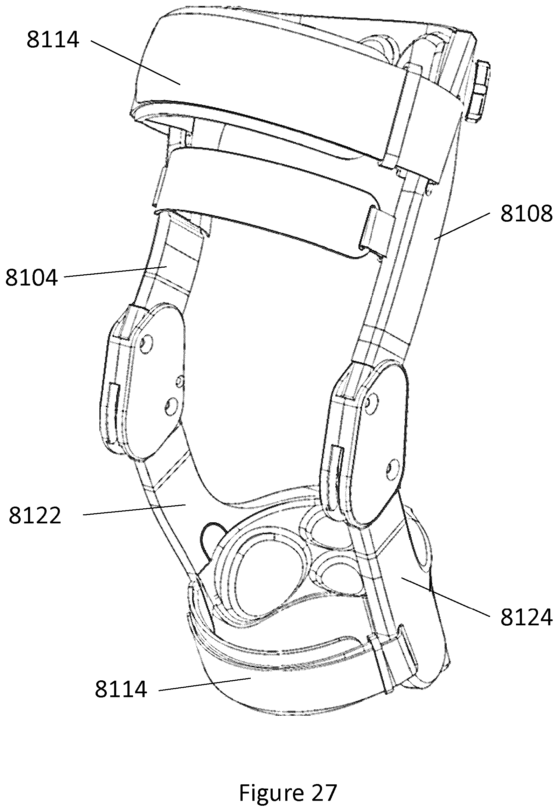

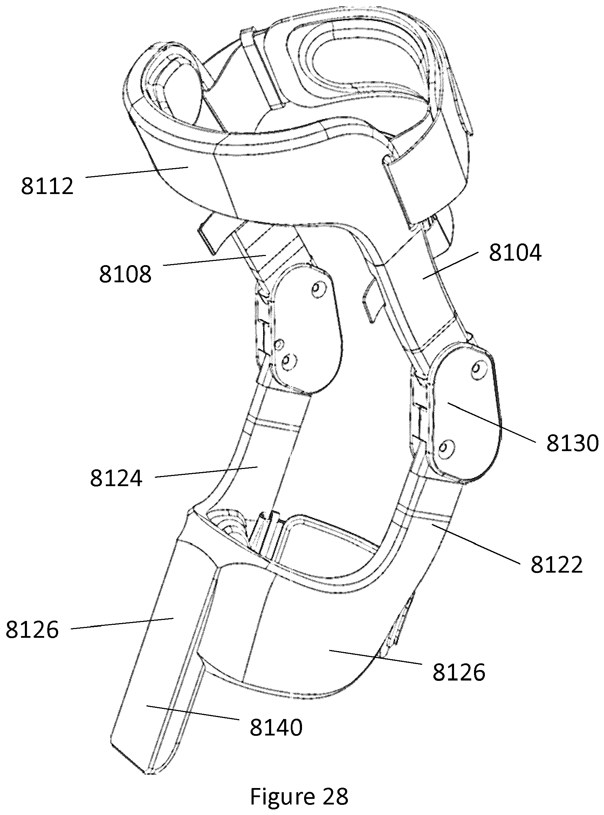

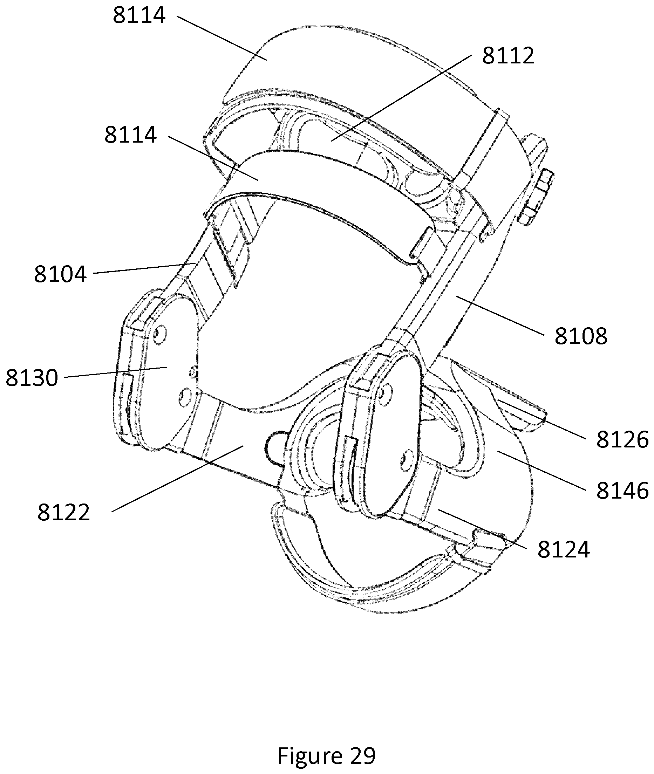

As noted previously, in some applications it may be desirable to configure the brace so that its cross-members are only provided on the posterior side of the upper frame and the posterior side of the lower frame. Also, it may be preferable in some situations to position the energy storage assembly (and preferably at least the spring member) on the posterior side of the brace, and optionally on the lower frame of the brace (so that it overlies a user's calf) rather than on the upper frame (where the bulk of the energy storage assembly could interfere with a user sitting down, etc. while wearing the brace). Referring to Figures 26-29, another example of a brace 8100 is illustrated. The brace 8100 in this example is generally similar to the brace 100, and like features are annotated using like reference characters indexed by 8000. In this example, the brace 8100 includes upper and lower frames 8102 and 8120, each having respective arms 8104, 8108, 8122 and 8124 and cross-members 8112 and 8126 that are position on the rear/ posterior side of the brace 8100 instead of on the front. Fastening straps 8114 are provided to help secure the upper and lower frames 8102 and 8120 to the user's leg.

The upper and lower frames 8102 and 8120 are connected by hinges 8130, and an energy storage assembly 8140 is mounted to the lower cross-member 8126 and is positioned on the rear of the brace 8100. A spring member 8146 is contained within the energy storage assembly 8140, and can include any suitable example of a spring, including the compression springs with guide pulleys and other features as illustrated in other embodiments herein.

In this arrangement, a tensioning cord can run through the brace 8100 as described herein, but the cord is not visible from the outside of brace 8100 as illustrated in these figures. However, in this arrangement the anchor portions of the cord (analogous to anchor portions 170) are located within the upper frame 8120 (rather than lower frame) and can be anchored within the arms 8104 and 8108 or, if the cord is continuous, to secure the cord to the upper frame the anchor portions can be wrapped around the rotatable spool/spindle 8220 of a winding apparatus 8222. In this arrangement, turning the spindle 1220 in one direction can wind the cord around the spindle 8220, which has the effect of drawing portions of the cord upwardly toward the apparatus 8222, thereby increasing the tension along the entire length of the cord. Depending on the tightness, winding the cord in this manner may constrict the bight region within the energy storage assembly 8140 and may pre-load the springs. Turning the spindle 8220 in the opposite direction can unwind the cord, thereby loosening the cord and reducing the tension in the cord. Once the cord is in the desired position the user can lock or otherwise immobilize the winding apparatus 8222, which can fix the anchor sections in a set position relative to the upper frame 8102. With the winding apparatus 8222 locked, the anchor sections do not translate relative to the upper frame 8120, and the remaining portions of the cord can operate as described herein, so that flexing the brace 8100 loads the springs. The winding apparatus 8222 can be any suitable type of mechanism, including a cord winding ratcheting pawl system (such as a BOA^{®}).

From the upper anchor portions, the cord in the brace 8100 can pass through the hinges 8130 and into the energized cord path (analogous to the energized cord paths 176) that extends, in this example, form the hinge 8130, through the lower arms 8122 and 8124 to the energy storage assembly 8140. The portion or segment of the cord within these lower, energized cord paths (e.g. extending form the hinge 8130 to at least the energy storage assembly 8140 when the brace 8100 is extended) can translate within the energized cord path when the brace 8100 is flexed and extended. The cord path within the energy storage assembly 8140 can be analogous to those described herein, and can be looped to provide a bight that surrounds the contained hydraulic springs and that constricts when the brace is flexed - or when the brace 8100 is moved from its neutral position to a loaded position.

While the embodiments herein have been primarily illustrated as being applied to knee braces for humans, the principles and features of the braces described herein can also be used on other types of brace, such as elbow braces for humans. The principles of teachings herein can also be applied to braces for animals including (without limitation) horses, dogs and cats It will also be appreciated that the energy storage features of the present teachings can advantageously be used in many other applications and the principles of the invention will apply equally. It will further be appreciated that all the advantages of the teachings herein do not necessarily apply to every embodiment.

## Claims

1. An energy storing knee brace (100) to be worn on a leg, the knee brace comprising:
a) an upper frame (102) having first and second longitudinally extending upper arms (102, 108) and an upper cross-member (112) extending laterally there between, the upper frame (102) configured to engage a user's leg above a knee joint;
b) a lower frame (120) having first and second longitudinally extending lower arms (122, 124) and a lower cross-member extending laterally there between, the lower frame (120) configured to engage the user's leg below the knee joint;
c) a first hinge (130) pivotally connecting the first upper arm (102) to the first lower arm (122), and comprising a first extension member having a first peripheral surface;
d) a second hinge (130) pivotally connecting the second upper arm (108) to the second lower arm (124), and comprising a second extension member having a second peripheral surface;
e) an energy storage assembly (140) mounted on the upper frame (102) or the lower frame (120) and comprising a spring member (146) that is spaced apart from the first hinge (130) and the second hinge (130);
f) a first energized cord path (176) extending from the first hinge (130) to the energy storage assembly (140), and a second energized cord path (176) extending from the second hinge (130) to the energy storage assembly (140);
g) a flexible tensioning cord (160) having
i. a first cord segment extending from a first anchor section secured relative to (a) the first lower arm when the energy storage assembly is mounted on the upper frame or (b) the first upper arm when the energy storage assembly is mounted on the lower frame, across the first peripheral surface and through the first energized cord path (176) to the spring member (146),
ii. a second cord segment extending from a second anchor section secured relative to (a) the second lower arm when the energy storage assembly is mounted on the upper frame or (b) the second upper arm when the energy storage assembly is mounted on the lower frame, across the second peripheral surface and through the second energized cord path (176) to the spring member (146);
wherein the first and second peripheral surfaces are configured so that pivoting the brace (100) from an extended position toward a flexed position causes (i) the first extension member to bear against and exert a tension force on the first cord segment thereby drawing the first cord segment through the first energized cord path (176) and away from the energy storage assembly (140), and (ii) the second extension member to bear against and exert a tension force on the second cord segment thereby drawing the second cord segment through the second energized cord path (176) and away from the energy storage assembly (140), thereby loading the spring member (146) and whereby the spring member (146) applies a restorative spring force on the first and second peripheral surfaces via the tensioning cord urging the brace to return to the extended position;
**characterised in that**
the tensioning cord (160) is looped around the spring member (146) so that the spring member (146) is disposed within a loop of the tensioning cord (160) and the tensioning cord comprises a spring portion (196) that engages a first end of the spring member (146), and wherein the tension force applied to the tensioning cord (160) when pivoting the brace from the extended position toward the flexed position causes the loop to constrict thereby urging the spring portion (196) to compress the spring member (146) and loading the spring member (146).

2. The knee brace (100) of claim 1, wherein the first cord segment comprises a first longitudinally oriented section extending from the first hinge along the first upper arm on a first side of the spring member, a second longitudinally oriented section extending from the spring portion (196) and a laterally extending transverse section connecting the first and second longitudinally oriented sections.

3. The knee brace (100) of claim 1 or claim 2, wherein the spring member (146) comprises at least a first hydraulic compression spring (148) configured so that exerting the tension force on the tensioning cord (160) compresses the first hydraulic compression spring (148), thereby loading the spring member (146)

4. The knee brace (100) of claim 3, further comprising a cord guide located proximate a second end of the spring member and configured to redirect the laterally extending transverse section of the first cord segment toward the second longitudinally oriented section of the first cord segment, and
wherein the cord guide comprises a first guide member proximate the second end of the spring member and a second guide member laterally offset from the first guide member, between the first guide member and the second upper arm, and wherein the first cord segment is routed such that it exerts a force on the first guide member acting toward the spring member and an opposing force on the second guide member acting away from the spring member, and
wherein the first guide member comprises a first pulley and the second guide member comprises a second pulley and wherein the first cord segment is received in a first groove on the first pulley and a first groove on the second pulley, and wherein the second cord segment is received in a second groove on the first pulley that is parallel to and offset from the first groove on the first pulley and a second groove on the second pulley that is parallel to and offset from the first groove on the second pulley.

5. The knee brace (100) of claim 3 or 4, wherein the restorative spring force and an opposing grounding force exerted by the spring member (146) are each carried by the tensioning cord (160), whereby the spring member (146) is compressed without exerting a substantial grounding force on the upper frame (102) when the energy storage assembly is mounted to the upper frame (102).

6. The knee brace (100) of any one of claims 3 to 5, wherein the spring member (146) comprises the first hydraulic compression spring (148) and a second hydraulic compression spring (148) arranged in parallel with the first hydraulic compression spring (148).

7. The knee brace (100) of claim 6, wherein:
a) the first hydraulic tension spring (148) comprises a first cylinder (150) containing a compressible hydraulic fluid, and a first piston slidably received within the first cylinder (150) and having a first piston rod (152) with a first outer end (156) that is disposed outside the first cylinder (150) and engages the spring portion (196) of the tensioning cord (160); and
b) the second hydraulic tension spring (148) comprises a second cylinder (150) that is parallel to the first cylinder (150) and contains a compressible hydraulic fluid, and a second piston slidably received within the second cylinder (150) and having a second piston rod (152) that is parallel to the first piston rod (152) and having a second outer end (156) that is disposed outside the second cylinder (150) and engages the spring portion (196) of the tensioning cord (160).

8. The knee brace (100) of claim 7, wherein the first outer end (156) is connected to the second outer end (156) by a bridge (158), whereby the first piston rod (152) and second piston rod (152) slide in unison.

9. The knee brace (100) of claim 7 or 8, wherein the spring portion (196) of the tensioning cord (160) can translate relative to the first outer end (156) and the second outer end (156) when the knee brace (100) is flexed thereby balancing the tension in the first cord segment and the second cord segment.

10. The knee brace (100) of any one of claims 1 to 9, wherein the first energized cord path has a longitudinal segment extending along the first upper arm and a lateral segment extending longitudinally from the first upper arm (102) to the energy storage assembly.

11. The knee brace (100) of claim 10, wherein the first cord segment is connected to the second energized cord segment and the first anchor segment is connected to the second anchor segment such that the tensioning cord (160) comprises a generally continuous loop, extending through the upper and lower frames (102, 120) the energy storage assembly (140) and the first and second hinges (130).

12. The knee brace (100) of claim 10 or claim 11, wherein the energy storage assembly (140) comprises a housing (144) at least partially surrounding the spring member (146), and wherein the first upper arm (102), the second upper arm (108), the upper cross-member and the housing (144) are of integral, one-piece construction.

13. The knee brace (100) of claim 10, further comprising a tension adjustment mechanism that includes a rotatable spindle connected to the lower frame (120), and wherein the tensioning cord (160) is connected to the spindle to that turning the spindle in one direction can wind the tensioning cord (160) around the spindle thereby drawing portions of the tensioning cord away from the energy storage assembly and increasing the tension along the entire length of the tensioning cord, and turning the spindle in an opposite direction can unwind the tensioning cord around the spindle, thereby allowing portions of the tensioning cord to be drawn toward the energy storage assembly and decreasing the tension along the entire length of the tensioning cord.

14. The knee brace (100) of claim 1, wherein the energy storage assembly (140) is mounted on the lower cross-member; and optionally wherein the lower cross-member is on a posterior side of the lower frame (120).

15. The knee brace of claim 1, wherein the energy storage assembly (140) is mounted on the lower frame (120) and the upper cross-member and lower cross-member are both disposed on the same one of an anterior and a posterior side of the knee brace (100).

## Patentansprüche

1. Energiespeichernde Kniestütze (100), die an einem Bein getragen wird, wobei die Kniestütze Folgendes umfasst:
a) einen oberen Rahmen (102), der einen ersten und einen zweiten sich längs erstreckenden oberen Arm (102, 108) und ein oberes Querelement (112) aufweist, das sich seitwärts dazwischen erstreckt, wobei der obere Rahmen (102) dazu konfiguriert ist, ein Bein eines Benutzers oberhalb eines Kniegelenks in Eingriff zu nehmen;
b) einen unteren Rahmen (120), der einen ersten und einen zweiten sich längs erstreckenden unteren Arm (122, 124) und ein unteres Querelement aufweist, das sich seitwärts dazwischen erstreckt, wobei der untere Rahmen (120) dazu konfiguriert ist, das Bein des Benutzers unterhalb des Kniegelenks in Eingriff zu nehmen;
c) ein erstes Scharnier (130), das den ersten oberen Arm (102) schwenkbar mit dem ersten unteren Arm (122) verbindet und ein erstes Erstreckungselement umfasst, das eine erste Umfangsfläche aufweist;
c) ein zweites Scharnier (130), das den zweiten oberen Arm (108) schwenkbar mit dem zweiten unteren Arm (124) verbindet und ein zweites Erstreckungselement umfasst, das eine zweite Umfangsfläche aufweist;
e) eine Energiespeicheranordnung (140), die am oberen Rahmen (102) oder am unteren Rahmen (120) angebracht ist und ein Federelement (146) umfasst, das von dem ersten Scharnier (130) und dem zweiten Scharnier (130) beabstandet ist;
f) einen ersten unter Spannung stehenden Seilpfad (176), der sich vom ersten Scharnier (130) bis zur Energiespeicheranordnung (140) erstreckt, und einen zweiten unter Spannung stehenden Seilpfad (176), der sich vom zweiten Scharnier (130) bis zur Energiespeicheranordnung (140) erstreckt;
g) ein biegbares Spannseil (160), aufweisend
i. ein erstes Seilsegment, das sich von einem ersten Verankerungsabschnitt, der relativ zu (a) dem ersten unteren Arm befestigt ist, wenn die Energiespeicheranordnung am oberen Rahmen angebracht ist, oder (b) dem ersten oberen Arm befestigt ist, wenn die Energiespeicheranordnung am unteren Rahmen angebracht ist, über die erste Umfangsfläche und durch den ersten unter Spannung stehenden Seilpfad (176) zum Federelement (146) erstreckt,
ii. ein zweites Seilsegment, das sich von einem zweiten Verankerungsabschnitt, der relativ zu (a) dem zweiten unteren Arm befestigt ist, wenn die Energiespeicheranordnung am oberen Rahmen angebracht ist, oder (b) dem zweiten oberen Arm befestigt ist, wenn die Energiespeicheranordnung am unteren Rahmen angebracht ist, über die zweite Umfangsfläche und durch den zweiten unter Spannung stehenden Seilpfad (176) zum Federelement (146) erstreckt;
wobei die erste und die zweite Umfangsfläche so konfiguriert sind, dass ein Schwenken der Stütze (100) aus einer erstreckten Position in eine gebogene Position verursacht, dass (i) das erste Erstreckungselement gegen das erste Seilsegment drückt und eine Spannkraft auf dieses ausübt, wodurch das erste Seilsegment durch den ersten unter Spannung stehenden Seilpfad (176) und weg von der Energiespeicheranordnung (140) gezogen wird, und (ii) das zweite Erstreckungselement gegen das zweite Seilsegment drückt und eine Spannkraft auf dieses ausübt, wodurch das zweite Seilsegment durch den zweiten unter Spannung stehenden Seilpfad (176) und weg von der Energiespeicheranordnung (140) gezogen wird, wodurch das Federelement (146) belastet wird und wodurch das Federelement (146) über das Spannseil eine Rückstellfederkraft auf die erste und die zweite Umfangsfläche aufbringt, die die Stütze in die erstreckte Position zurückdrängt;
**dadurch gekennzeichnet, dass**
das Spannseil (160) um das Federelement (146) eine Schleife gemacht hat, sodass das Federelement (146) innerhalb einer Schleife des Spannseils (160) angeordnet ist, und das Spannseil einen Federteil (196) umfasst, der mit einem ersten Ende des Federelements (146) in Eingriff steht, und wobei die beim Schwenken der Stütze aus der erstreckten Position in die gebogene Position auf das Spannseil (160) aufgebrachte Spannkraft verursacht, dass sich die Schleife zusammenzieht, wodurch der Federteil (196) gedrängt wird, das Federelement (146) zusammenzudrücken und das Federelement (146) zu belasten.

2. Kniestütze (100) nach Anspruch 1, wobei das erste Seilsegment einen ersten längsorientierten Abschnitt, der sich vom ersten Scharnier entlang des ersten oberen Arms auf einer ersten Seite des Federelements erstreckt, einen zweiten längsorientierten Abschnitt, der sich vom Federteil (196) erstreckt, und einen sich seitwärts erstreckenden transversalen Abschnitt umfasst, der den ersten und zweiten längsorientierten Abschnitt verbindet.

3. Kniestütze (100) nach Anspruch 1 oder Anspruch 2, wobei das Federelement (146) mindestens eine erste hydraulische Druckfeder (148) umfasst, die so konfiguriert ist, dass durch Ausüben der Spannkraft auf das Spannseil (160) die erste hydraulische Druckfeder (148) zusammengedrückt, wodurch das Federelement (146) belastet wird.

4. Kniestütze (100) nach Anspruch 3, weiter umfassend eine Seilführung, die sich in der Nähe eines zweiten Endes des Federelements befindet und dazu konfiguriert ist, den sich seitwärts erstreckenden transversalen Abschnitt des ersten Seilsegments in Richtung des zweiten längsorientierten Abschnitts des ersten Seilsegments umzulenken, und
wobei die Seilführung ein erstes Führungselement in der Nähe des zweiten Endes des Federelements und ein zweites Führungselement umfasst, das seitwärts vom ersten Führungselement versetzt ist, zwischen dem ersten Führungselement und dem zweiten oberen Arm, und wobei das erste Seilsegment so geführt ist, dass es eine Kraft auf das erste Führungselement ausübt, die in Richtung des Federelements wirkt, und eine entgegengesetzte Kraft auf das zweite Führungselement ausübt, die vom Federelement weg wirkt, und
wobei das erste Führungselement eine erste Rolle umfasst und das zweite Führungselement eine zweite Rolle umfasst und wobei das erste Seilsegment in einer ersten Nut an der ersten Rolle und einer ersten Nut an der zweiten Rolle aufgenommen ist, und wobei das zweite Seilsegment in einer zweiten Nut an der ersten Rolle, die parallel zu der ersten Nut an der ersten Rolle ist und von dieser versetzt ist, und in einer zweiten Nut an der zweiten Rolle, die parallel zu der ersten Nut an der zweiten Rolle ist und von dieser versetzt ist, aufgenommen ist.

5. Kniestütze (100) nach Anspruch 3 oder 4, wobei die Rückstellfederkraft und eine entgegenwirkende Erdungskraft, die vom Federelement (146) ausgeübt werden, jeweils vom Spannseil (160) getragen werden, wodurch das Federelement (146) zusammengedrückt wird, ohne eine wesentliche Erdungskraft auf den oberen Rahmen (102) auszuüben, wenn die Energiespeicheranordnung am oberen Rahmen (102) angebracht ist.

6. Kniestütze (100) nach einem der Ansprüche 3 bis 5, wobei das Federelement (146) die erste hydraulische Druckfeder (148) und eine zweite hydraulische Druckfeder (148) umfasst, die parallel zur ersten hydraulischen Druckfeder (148) eingerichtet ist.

7. Kniestütze (100) nach Anspruch 6, wobei:
a) die erste hydraulische Zugfeder (148) einen ersten Zylinder (150), der ein zusammendrückbares Hydraulikfluid enthält, und einen ersten Kolben, der gleitend innerhalb des ersten Zylinders (150) aufgenommen ist und eine erste Kolbenstange (152) mit einem ersten äußeren Ende (156) aufweist, das außerhalb des ersten Zylinders (150) angeordnet ist und mit dem Federteil (196) des Spannseils (160) eingreift; und
b) die zweite hydraulische Zugfeder (148) einen zweiten Zylinder (150), der parallel zum ersten Zylinder (150) ist und ein zusammendrückbares Hydraulikfluid enthält, und einen zweiten Kolben, der gleitend innerhalb des zweiten Zylinders (150) aufgenommen ist und eine zweite Kolbenstange (152) aufweist, die parallel zur ersten Kolbenstange (152) ist, und ein zweites äußeres Ende (156) aufweist, das außerhalb des zweiten Zylinders (150) angeordnet ist und mit dem Federteil (196) des Spannseils (160) eingreift.

8. Kniestütze (100) nach Anspruch 7, wobei das erste äußere Ende (156) mit dem zweiten äußeren Ende (156) über eine Brücke (158) verbunden ist, wodurch die erste Kolbenstange (152) und die zweite Kolbenstange (152) synchron gleiten.

9. Kniestütze (100) nach Anspruch 7 oder 8, wobei sich der Federteil (196) des Spannseils (160) relativ zum ersten äußeren Ende (156) und zum zweiten äußeren Ende (156) verschieben kann, wenn die Kniestütze (100) gebogen wird, wodurch die Spannung im ersten Seilsegment und im zweiten Seilsegment ausgeglichen wird.

10. Kniestütze (100) nach einem der Ansprüche 1 bis 9, wobei der erste unter Spannung stehende Seilpfad ein Längssegment, das sich entlang des ersten oberen Arms erstreckt, und ein Seitensegment aufweist, das sich längs vom ersten oberen Arm (102) zur Energiespeicheranordnung erstreckt.

11. Kniestütze (100) nach Anspruch 10, wobei das erste Seilsegment mit dem zweiten unter Spannung stehenden Seilsegment verbunden ist und das erste Ankersegment mit dem zweiten Ankersegment verbunden ist, sodass das Spannseil (160) eine im Wesentlichen durchgehende Schleife umfasst, die sich durch den oberen und unteren Rahmen (102, 120), die Energiespeicheranordnung (140) und das erste und zweite Scharnier (130) erstreckt.

12. Kniestütze (100) nach Anspruch 10 oder Anspruch 11, wobei die Energiespeicheranordnung (140) ein Gehäuse (144) umfasst, das das Federelement (146) mindestens teilweise umschließt, und wobei der erste obere Arm (102), der zweite obere Arm (108), das obere Querelement und das Gehäuse (144) von ganzheitlicher, einteiliger Konstruktion ist.

13. Kniestütze (100) nach Anspruch 10, die weiter einen Spannungsregulierungsmechanismus umfasst, der eine drehbare Spindel beinhaltet, die mit dem unteren Rahmen (120) verbunden ist, und wobei das Spannseil (160) mit der Spindel verbunden ist, sodass durch Drehen der Spindel in eine Richtung das Spannseil (160) um die Spindel gewickelt wird, wodurch Teile des Spannseils von der Energiespeicheranordnung weggezogen und die Spannung über die gesamte Länge des Spannseils erhöht wird, und durch Drehen der Spindel in die entgegengesetzte Richtung das Spannseil um die Spindel abgewickelt wird, wodurch Teile des Spannseils zur Energiespeicheranordnung hingezogen werden können und die Spannung über die gesamte Länge des Spannseils verringert wird.

14. Kniestütze (100) nach Anspruch 1, wobei die Energiespeicheranordnung (140) am unteren Querelement angebracht ist; und wobei sich das untere Querelement optional auf einer posterioren Seite des unteren Rahmens (120) befindet.

15. Kniestütze nach Anspruch 1, wobei die Energiespeicheranordnung (140) am unteren Rahmen (120) angebracht ist und sowohl das obere Querelement als auch das untere Querelement auf derselben einer anterioren und einer posterioren Seite der Kniestütze (100) angeordnet sind.

## Revendications

1. Attelle de genou à stockage d'énergie (100) à porter sur une jambe, l'attelle de genou comprenant :
a) un cadre supérieur (102) présentant un premier et un second bras supérieurs s'étendant longitudinalement (102, 108) et une traverse supérieure (112) s'étendant latéralement entre eux, le cadre supérieur (102) étant configuré pour engager la jambe d'un utilisateur au-dessus d'une articulation du genou ;
b) un cadre inférieur (120) présentant un premier et un second bras inférieurs s'étendant longitudinalement (122, 124) et une traverse inférieure s'étendant latéralement entre eux, le cadre inférieur (120) étant configuré pour engager la jambe de l'utilisateur sous l'articulation du genou ;
c) une première charnière (130) connectant de manière pivotante le premier bras supérieur (102) au premier bras inférieur (122), et comprenant un premier élément d'extension présentant une première surface périphérique ;
d) une seconde charnière (130) connectant de manière pivotante le second bras supérieur (108) au second bras inférieur (124), et comprenant un second élément d'extension présentant une seconde surface périphérique ;
e) un ensemble de stockage d'énergie (140) monté sur le cadre supérieur (102) ou le cadre inférieur (120) et comprenant un élément de ressort (146) qui est espacé de la première charnière (130) et de la seconde charnière (130) ;
f) un premier chemin de cordon alimenté (176) s'étendant de la première charnière (130) à l'ensemble de stockage d'énergie (140), et un second chemin de cordon alimenté (176) s'étendant de la seconde charnière (130) à l'ensemble de stockage d'énergie (140) ;
g) un cordon de mise en tension flexible (160) présentant
i. un premier segment de cordon s'étendant d'une première section d'ancrage fixée par rapport à (a) le premier bras inférieur lorsque l'ensemble de stockage d'énergie est monté sur le cadre supérieur ou (b) le premier bras supérieur lorsque l'ensemble de stockage d'énergie est monté sur le cadre inférieur, à travers la première surface périphérique et à travers le premier chemin de cordon alimenté (176) jusqu'à l'élément de ressort (146),
ii. un second segment de cordon s'étendant d'une seconde section d'ancrage fixée par rapport à (a) le second bras inférieur lorsque l'ensemble de stockage d'énergie est monté sur le cadre supérieur ou (b) le second bras supérieur lorsque l'ensemble de stockage d'énergie est monté sur le cadre inférieur, à travers la seconde surface périphérique et à travers le second chemin de cordon alimenté (176) jusqu'à l'élément de ressort (146) ;
dans laquelle les première et seconde surfaces périphériques sont configurées de sorte que le pivotement de l'attelle (100) d'une position étendue vers une position fléchie amène (i) le premier élément d'extension à appuyer contre et exercer une force de tension sur le premier segment de cordon, tirant ainsi le premier segment de cordon à travers le premier chemin de cordon alimenté (176) et à l'écart de l'ensemble de stockage d'énergie (140), et (ii) le second élément d'extension à appuyer contre et exercer une force de tension sur le second segment de cordon, tirant ainsi le second segment de cordon à travers le second chemin de cordon alimenté (176) et à l'écart de l'ensemble de stockage d'énergie (140), chargeant ainsi l'élément de ressort (146) et moyennant quoi l'élément de ressort (146) applique une force de ressort de restauration sur les première et seconde surfaces périphériques par l'intermédiaire du cordon de mise en tension, incitant l'attelle à revenir à la position étendue ;
**caractérisée en ce que**
le cordon de mise en tension (160) est enroulé autour de l'élément de ressort (146) de sorte que l'élément de ressort (146) est disposé au sein d'une boucle du cordon de mise en tension (160) et le cordon de mise en tension comprend une portion de ressort (196) qui engage une première extrémité de l'élément de ressort (146), et dans laquelle la force de tension appliquée au cordon de mise en tension (160) lors du pivotement de l'attelle de la position étendue vers la position fléchie amène la boucle à se resserrer, incitant ainsi la portion de ressort (196) à comprimer l'élément de ressort (146) et à charger l'élément de ressort (146).

2. Attelle de genou (100) selon la revendication 1, dans laquelle le premier segment de cordon comprend une première section orientée longitudinalement s'étendant de la première charnière le long du premier bras supérieur sur un premier côté de l'élément de ressort, une seconde section orientée longitudinalement s'étendant de la portion de ressort (196) et une section transversale s'étendant latéralement connectant les première et seconde sections orientées longitudinalement.

3. Attelle de genou (100) selon la revendication 1 ou la revendication 2, dans laquelle l'élément de ressort (146) comprend au moins un premier ressort de compression hydraulique (148) configuré de sorte que l'exercice de la force de tension sur le cordon de mise en tension (160) comprime le premier ressort de compression hydraulique (148), chargeant ainsi l'élément de ressort (146)

4. Attelle de genou (100) selon la revendication 3, comprenant en outre un guide de cordon situé à proximité d'une seconde extrémité de l'élément de ressort et configuré pour rediriger la section transversale s'étendant latéralement du premier segment de cordon vers la seconde section orientée longitudinalement du premier segment de cordon, et
dans laquelle le guide de cordon comprend un premier élément de guide à proximité de la seconde extrémité de l'élément de ressort et un second élément de guide décalé latéralement par rapport au premier élément de guide, entre le premier élément de guide et le second bras supérieur, et dans laquelle le premier segment de cordon est acheminé de sorte qu'il exerce une force sur le premier élément de guide agissant vers l'élément de ressort et une force opposée sur le second élément de guide agissant à l'écart de l'élément de ressort, et
dans laquelle le premier élément de guide comprend une première poulie et le second élément de guide comprend une seconde poulie, et dans laquelle le premier segment de cordon est reçu dans une première rainure de la première poulie et dans une première rainure de la seconde poulie, et dans laquelle le second segment de cordon est reçu dans une seconde rainure de la première poulie qui est parallèle et décalée par rapport à la première rainure sur la première poulie, et dans une seconde rainure de la seconde poulie qui est parallèle et décalée par rapport à la première rainure sur la seconde poulie.

5. Attelle de genou (100) selon la revendication 3 ou 4, dans laquelle la force de ressort de restauration et une force de mise à la terre opposée exercée par l'élément de ressort (146) sont chacune supportées par le cordon de mise en tension (160), moyennant quoi l'élément de ressort (146) est comprimé sans exercer une force de mise à la terre substantielle sur le cadre supérieur (102) lorsque l'ensemble de stockage d'énergie est monté sur le cadre supérieur (102).

6. Attelle de genou (100) selon l'une quelconque des revendications 3 à 5, dans laquelle l'élément de ressort (146) comprend le premier ressort de compression hydraulique (148) et un second ressort de compression hydraulique (148) agencé en parallèle avec le premier ressort de compression hydraulique (148).

7. Attelle de genou (100) selon la revendication 6, dans laquelle :
a) le premier ressort de tension hydraulique (148) comprend un premier cylindre (150) contenant un fluide hydraulique compressible, et un premier piston coulissant reçu au sein du premier cylindre (150) et présentant une première tige de piston (152) avec une première extrémité extérieure (156) qui est disposée à l'extérieur du premier cylindre (150) et engage la portion de ressort (196) du câble de mise en tension (160) ; et
b) le second ressort de tension hydraulique (148) comprend un second cylindre (150) qui est parallèle au premier cylindre (150) et contient un fluide hydraulique compressible, et un second piston coulissant reçu au sein du second cylindre (150) et présentant une seconde tige de piston (152) qui est parallèle à la première tige de piston (152) et présentant une seconde extrémité extérieure (156) qui est disposée à l'extérieur du second cylindre (150) et engage la portion de ressort (196) du cordon de mise en tension (160).

8. Attelle de genou (100) selon la revendication 7, dans laquelle la première extrémité extérieure (156) est connectée à la seconde extrémité extérieure (156) par un pont (158), moyennant quoi la première tige de piston (152) et la seconde tige de piston (152) glissent à l'unisson.

9. Attelle de genou (100) selon la revendication 7 ou 8, dans laquelle la portion de ressort (196) du cordon de mise en tension (160) peut se déplacer par rapport à la première extrémité extérieure (156) et à la seconde extrémité extérieure (156) lorsque l'attelle de genou (100) est fléchie, équilibrant ainsi la tension dans le premier segment de cordon et le second segment de cordon.

10. Attelle de genou (100) selon l'une quelconque des revendications 1 à 9, dans laquelle le premier chemin de cordon alimenté présente un segment longitudinal s'étendant le long du premier bras supérieur et un segment latéral s'étendant longitudinalement du premier bras supérieur (102) à l'ensemble de stockage d'énergie.

11. Attelle de genou (100) selon la revendication 10, dans laquelle le premier segment de cordon est connecté au second segment de cordon alimenté et le premier segment d'ancrage est connecté au second segment d'ancrage de sorte que le cordon de mise en tension (160) comprend une boucle généralement continue, s'étendant à travers les cadres supérieur et inférieur (102, 120), l'ensemble de stockage d'énergie (140) et les première et seconde charnières (130).

12. Attelle de genou (100) selon la revendication 10 ou la revendication 11, dans laquelle l'ensemble de stockage d'énergie (140) comprend un boîtier (144) entourant au moins partiellement l'élément de ressort (146), et dans laquelle le premier bras supérieur (102), le second bras supérieur (108), la traverse supérieure et le boîtier (144) sont de construction monobloc d'un seul tenant.

13. Attelle de genou (100) selon la revendication 10, comprenant en outre un mécanisme d'ajustement de tension qui inclut une broche rotative connectée au cadre inférieur (120), et dans laquelle le cordon de mise en tension (160) est connecté à la broche de sorte que la rotation de la broche dans une direction peut enrouler le cordon de mise en tension (160) autour de la broche, tirant ainsi des portions du cordon de mise en tension à l'écart de l'ensemble de stockage d'énergie et augmentant la tension sur toute la longueur du cordon de mise en tension, et la rotation de la broche dans la direction opposée peut dérouler le cordon de mise en tension autour de la broche, permettant ainsi à des portions du cordon de mise en tension d'être tirées vers l'ensemble de stockage d'énergie et diminuant la tension sur toute la longueur du cordon de mise en tension.

14. Attelle de genou (100) selon la revendication 1, dans laquelle l'ensemble de stockage d'énergie (140) est monté sur la traverse inférieure ; et facultativement dans laquelle la traverse inférieure est sur un côté postérieur du cadre inférieur (120).

15. Attelle de genou selon la revendication 1, dans laquelle l'ensemble de stockage d'énergie (140) est monté sur le cadre inférieur (120) et la traverse supérieure et la traverse inférieure sont toutes deux disposées du même côté antérieur et postérieur de l'attelle de genou (100).
